(19) Europäisches Patentamt / European Patent Office / Office européen des brevets

(11) **EP 4 544 999 A1**

(12) **EUROPEAN PATENT APPLICATION**
published in accordance with Art. 153(4) EPC

(43) Date of publication:
**30.04.2025 Bulletin 2025/18**

(21) Application number: 23827247.0

(22) Date of filing: **21.06.2023**

(51) International Patent Classification (IPC):
*A61B 5/107* (2006.01)     *A61B 5/00* (2006.01)
*A61B 5/0533* (2021.01)     *A61B 5/113* (2006.01)
*H10N 30/30* (2023.01)     *H10N 30/857* (2023.01)

(52) Cooperative Patent Classification (CPC):
A61B 5/00; A61B 5/0533; A61B 5/107;
A61B 5/113; H10N 30/30; H10N 30/857

(86) International application number:
**PCT/JP2023/022982**

(87) International publication number:
**WO 2023/249064 (28.12.2023 Gazette 2023/52)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **22.06.2022 JP 2022100690**

(71) Applicants:
• **Asahi Kasei Advance Corporation**
  **Tokyo 105-0004 (JP)**
• **Osaka Research Institute of Industrial Science and Technology**
  **Izumi-shi, Osaka 594-1157 (JP)**

(72) Inventors:
• **MORITA, Shigeru**
  **Tokyo 100-0006 (JP)**

• **SAITO, Daisuke**
  **Tokyo 100-0006 (JP)**
• **UNO, Mayumi**
  **Izumi-shi, Osaka 594-1157 (JP)**
• **OMORI, Mariko**
  **Izumi-shi, Osaka 594-1157 (JP)**
• **YOSHIMURA, Kansei**
  **Kanazawa-shi, Ishikawa 920-3111 (JP)**
• **TSURUMI, Takuto**
  **Kanazawa-shi, Ishikawa 920-3111 (JP)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(54) **BIOMETRIC INFORMATION MEASUREMENT DEVICE**

(57)     Provided is a biometric information measurement device that can detect a change in the biometric information of a wearer (a movement of a finger, the bending and stretching of an arm or foot, a posture change during sitting, a change in length of the body surface due to respiration, and a change in surface moisture amount). The present invention relates to a biometric information measurement device that is made of a fiber base material including a sensing member, and that is disposed in contact with a prescribed position of a living body, wherein the sensing member has at least two core-sheath structure threads in which high-resistance fibers as a cladding material are disposed around a linear conductor as a core material, two among the at least two core-sheath structure threads are disposed adjacent to each other to make it possible to read a change in resistance and/or a change in capacitance between the linear conductors of the two core-sheath structure threads, and the biometric information or a fluctuation thereof is measured based on said change. The present invention also relates to a garment provided with said device.

EP 4 544 999 A1

# Fig. 2

Load

$C \pm \triangle C$

$R \mp \triangle R$

## Description

FIELD

The present invention relates to a biometric information measurement device. More specifically, the invention relates to a biometric information measurement device comprising a fiber base material that includes a sensing member having core-sheath structure yarn comprising high resistance fiber arranged as a covering material around a linear conductor as a core material, and is disposed in contact with a predetermined position of the human body, wherein the sensing member has at least two core-sheath structure yarns comprising high resistance fiber arranged as covering material around the linear conductor as the core material, two of which are disposed in mutual proximity, and is able to read off the change in resistance and/or change in capacitance between linear conductors of two core-sheath structured yarns, and based on that change, measure biometric information by detecting fluctuation in the predetermined position of the body or change in moisture content on a biological surface.

BACKGROUND

Smart textiles have been proposed in the prior art, having electrical functional elements formed on flexible or stretchable fiber base materials. Such textiles comprise functional elements such as a sensor, battery, heater and Peltier element provided on a flexible fiber base material, thus allowing realization of highly thin and flexible products which are considered extremely important for the future IoT (Internet of Things) society.

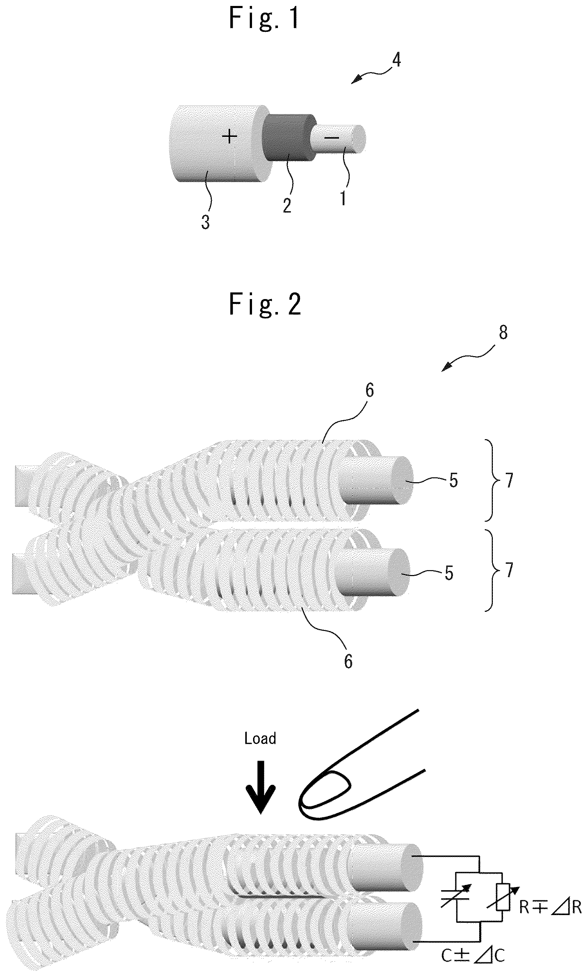

One type of such a sensor that has been proposed is a piezoelectric processed yarn or piezoelectric sensor such as shown in Fig. 1, being a filament member having a contact-sensing function. As described in PTLs 1 to 5, piezoelectric processed yarn (also referred to as piezoelectric thread or a piezoelectric filament) generally has a structure with conductive fibers wrapped by a piezoelectric material such as polylactic acid or polyvinylidene fluoride, and further wrapped with a covering of a conductor such as metal plating. Polylactic acid, a crystalline helical chiral polymer, exhibits a piezoelectric property when provided as a uniaxially stretched film. A piezoelectric property is the property whereby an electrical charge is generated upon application of stress. Thus, between the conductive fibers (1) on the inner layer side and the conductive fibers (3) on the outer side, for example, a "-" charge is generated on side (1) and a "+" charge is generated on side (3), as shown in Fig. 1. The opposite situation may also occur. It is therefore necessary to orient the piezoelectric material, which makes it difficult to increase productivity during the plating step, and therefore at the current time such materials are very costly at about 1000 to 5000 yen per 1 m of processed yarn. Moreover, with the structure shown in Fig. 1 it is currently difficult to produce processed yarn at lengths of 10,000 m or longer, thus also making it difficult to use piezoelectric threads as warp yarn for woven fabrics or warp knitting. The rigidity of polylactic acid as a piezoelectric material is also problematic since it causes the piezoelectric thread to have a poor feel and to lack flexibility for use as fiber.

Also known, as described in PTLs 6 and 7, are contact sensing fiber members that detect contact or load by detecting changes in electrostatic capacitance upon contact or load application between two adjacent electrodes, while techniques are also known for detecting changes in electrostatic capacitance produced when a conductor (such as the human body) approaches a single electrode. However the prior art mainly employs urethane or silicone insulators as insulators between electrode pairs, which makes it difficult to cause large variation in the distances between electrodes, thus lowering output (sensitivity), increasing cost, and resulting in an unsatisfactory feel. When a single electrode is used as described in PTL 8, the changes in electrostatic capacitance are extremely minute and can only be detected by highly advanced signal processing units, such that at the current time their contact sensitivity is low and their sensitivity as proximity sensors is also low.

Techniques relating to covering yarn with excellent bulkiness and highly productive and high-quality covering yarn are also known, as described in PTLs 9 and 10, but these publications do not teach or suggest the use of covering yarn techniques for sensing fiber members.

PTL 11 describes a device for monitoring respiration, the device being an article of clothing that can be worn by a user. The described device is a device for monitoring respiration by a user, comprising a fabric support which includes a cylindrical part formed by knitting electrically insulating ground yarn constituting the major portion, the cylindrical part being able to cover the chest of a user, at least one inner core which is at least one respiration sensor formed by knitting detection yarn, the detection yarn forming a plurality of stitches and the detection yarn being produced from an electrical insulating material, and at least one respiration sensor which includes an outer sheath surrounding the inner core, the outer sheath being produced from an electrically conductive material so as to form electrical contacts between the stitches of the detection yarn, wherein the respiration sensor forms a conductive band having a first end and a second end mutually separated by a distance, the ends being connectable to a device that measures the electrical resistance of the conductive band, the conductive band being disposed on the cylindrical part in such a manner that the conductive band undergoes alternating expansion and contraction due to respiration by the user when the chest of the user is covered by the fabric support, such expansion and contraction of the conductive band causing change in the electrical contacts between the stitches of the detection yarn in the conductive band, and thus creating change in the electrical resistance of the conductive band.

In PTL 11 it is stated that conventional elastic conductive yarn is obtained by surrounding a core composed of non-conductive elastic yarn with non-stretchable conductive yarn, or in other words, conductive yarn is wound in a spiral fashion around non-conductive elastic yarn, that stretching of the elastic conductive yarn causes the yarn to elongate, so that adjacent turns of the coils separate from each other and cause change in the measurability of the electrical resistance which depends on stretching of the elastic conductive yarn, and that surrounding conductive yarn with non-conductive yarn increases the total diameter of the conductive yarn, thereby increasing production cost for the clothing and potentially creating discomfort for the user who wears the clothing.

PTL 12 describes clothing for measurement of biometric information, a biometric information measurement system, a biometric information measuring device and a method for controlling the device, whereby biometric information for a subject can be precisely measured even with a simple construction. The resistance value sensor that is described detects fluctuation in the resistance values of a chest respiration information sensor and/or abdominal respiration information sensor, provided in the shirt part of a biometric information measuring shirt. An analysis device CPU calculates the fluctuation cycle for resistance based on the resistance value data, and outputs a respiration value based on the result. The clothing for measurement of biometric information is formed of a non-conductive material having stretchability allowing it to closely fit to the upper body of the subject, the clothing for measurement of biometric information including a conductive member that exhibits a variable electrical resistance value when electrified, in response to changes in shape with the differences in body shape that occur during breathing movement by the subject, and a respiration information measuring sensor unit being situated so as to allow transmission of electrical information based on changes in the electrical resistance value, to a respiration information analysis device. The biometric information measuring shirt has a chest electrode part and extremity electrode parts formed of a conductive material, knitted into the shirt part. An embodiment employs conductive fiber knitted into the back side of the shirt part (the side that contacts the skin of the subject), as an example for the chest electrode part and extremity electrode part. As an example of the conductive fiber, PTL 12 describes an embodiment using fibers with (chemical bond) adhering metal particles (such as silver particles, copper particles, or copper sulfide particles) (for example, THUNDERON[R] by Nihon Sanmo Dyeing Co., Ltd. The respiration information measuring sensor unit described is also placed at least around the chest or around the abdomen part of the clothing for measurement of biometric information, and exhibits a change in electrical resistance value in response to changes in cross-sectional area or length of the conductive member, due to stretching that occurs with differences in body shape caused by breathing movement by the subject. This feature allows the respiration information measuring sensor unit to directly detect physical changes in the circumference of the rib cage or the circumference of the abdomen (girth) due to breathing movement by the subject, for stable detection sensitivity of respiration information. The respiration information measuring sensor unit described has the surface of the conductive member that faces the body surface of the subject and its opposite surface covered with a non-conductive material, thus reducing electrical effects due to the state of electrification of the subject.

PTL 13 describes posture detecting clothing wherein a body fabric forming at least a front body region and a rear body region is composed of a stretch fabric that covers the body surface, the clothing being provided with a belt-shaped posture detecting stretching member which is disposed integrally with the body fabric forming the rear body region in a manner crossing with and bilaterally symmetrical with the center of the back, and which converts changes in the stretched state of the body fabric into changes in electrostatic capacitance, and being constructed so as to allow detection of hunched posture of the wearer based on changes in electrostatic capacitance detected by the posture detecting stretching member, wherein at least two posture detecting stretching members are disposed vertically across a spacing on the dorsal region of the rear body region when in the horizontal posture, each being made of a posture detecting knitted fabric whose electrostatic capacitance changes with stretching, corresponding to the stretched state of the body fabric, and integrally knitted with the body fabric or joined to the body fabric. The object of the invention as described is to provide posture detecting clothing that can be worn on a daily basis and allows objective detection and evaluation of the degree of bias of the posture of the wearer. The "conductive thread" is bare material with a metal component exposed on the thread surface. The "elastic yarn" is yarn of a material that maintains a contracted state in the absence of tensile load (when not stretched, or in the "normal state"), but freely stretches in response to tensile force when placed under a tensile load, and which is restored to the original contracted state from its stretched state (contracts) when the tensile force is released to return to an absence of load. The conductive thread used is preferably metal coated wire (plated wire) having a core of resin fiber or natural fiber, or metal wire, and with a metal component attached to the core by wet or dry coating, plating, vacuum film formation or another suitable method of attachment. The individual loops of the conductive thread are deformed into a contracted shape in the course direction and are kept in that deformed shape. Since the conductive thread is made of a conductive bare material, a greater number of contact points by the loops, and increased contact area by compression in the course direction, results in a greater number of conductive contact points, i.e. more conductive area, a shorter current path, and lower electrical resistance between any two separate points in the course direction.

PTL 14 describes a wearable device for detecting human body movement, which is a wearable device that is worn on part of the human body and detects action by the human body wearing it, the wearable device comprising an action detecting sensor unit that includes conductive thread and whose electrical characteristics change when stretched and when not stretched, and a warning information generating unit that generates warning-related information based on detection information from the action detecting sensor unit, wherein the action detecting sensor unit includes a conductive stretch knitted fabric whose electrical characteristics change when stretched and when not stretched and a fabric with a stretchable property on which the conductive stretch knitted fabric is attached, constructed in such a manner that the fabric has greater elongation resistance than the conductive stretch knitted fabric, where the fabric is formed as a long shape and constructed in a separable manner with a first base region having the warning information generating unit disposed along its lengthwise direction, and a second base region on which the conductive stretch knitted fabric is attached, and the first base region and second base region are constructed in a freely detachable manner using connecting means. The invention described is intended to provide a wearable device for detecting human body movement that, using a convenient construction, can precisely detect body movement information including human body movement at sites including bendable sites such as the joints or dorsal region of the human body, or respiration information, such as the presence or absence of breathing, or a cycle of respiration.

The conductive stretch knitted fabric described is a knitted fabric having high stretchability and flexibility and the ability to recover after repeated stretching, as well as variable electrical resistance when stretched and when not stretched, while also being able to exhibit gas permeability, moisture permeability and a water absorbing property, making it suitable for use as a wearable material. The conductive stretch knitted fabric has a simple construction and uses conductive stretchable yarn with variable electrical resistance in response to the elongation rate.

PTL 15 describes wear for detection of human body movement, which employs a conductive harness using a knitted fabric. Work gloves are an example of the wear for detection of human body movement described, and they are worn on human hands to detect movement of human fingers. The wear includes a gyro sensor module provided on the back hand sides of the work gloves corresponding to a first site of the human body, which detects a first physical quantity (angular velocity or angular acceleration) at the first site, and an acceleration sensor module provided on the thumb and index finger parts of the work gloves, corresponding to a second site via joints with the first site, which detects a second physical quantity (speed or acceleration) at the second site, and a conductive harness through which electrical signals outputted from the sensors flow, the conductive harness being bonded to the work gloves as wearable articles. The invention described is intended to provide wear for detection of human body movement, which has excellent stretchability and flexibility, and a recoverable property when subjected to repeated stretching, as an example of suitably applying a conductive harness that employs a conductive stretch knitted fabric exhibiting reduced or absolutely no change in electrical resistance even after repeated stretching.

PTL 16 describes a strain sensor-attached fabric that is unlikely to either impede movement of the wearer or cause wire breakage by movement of the wearer.

The strain sensor-attached fabric described has a stretchable fabric body and a strain sensor that is attached to the fabric body and is able to follow stretching of the fabric body, the strain sensor-attached fabric comprising a wiring part which is electrically connected to the strain sensor while being provided integrally with the fabric body, and deforming along with stretching of the fabric body. The wiring part can be formed by sewing a conductive filamentous body onto the fabric body. The wiring part can be formed by knitting or weaving a nonconductive filamentous body with a conductive filamentous body during knitting or weaving of the fabric body. The arranged section may be formed from a belt-shaped member attached to one side of the fabric body. The object of the invention described is to provide a strain sensor-attached fabric wherein the wiring does not interfere with movement that causes stretching of the fabric body and the wiring is resistant to breakage, and to provide clothing wherein movement by the wearer is unlikely to be impeded and wire breakage is unlikely to occur as a result of movement by the wearer.

The strain sensor-attached fabric may have a structure in which the wiring part is formed by sewing a conductive filamentous body onto the fabric body. This allows the wiring part to be easily and reliably formed by sewing of the filamentous body.

PTL 17 describes a motion measuring device that is provided on a sheet material worn on the human body and measures movement by the wearer, for the purpose of accurately detecting posture and movement of a wearer, such as hunching, while excluding the effects of fluctuation in the size (depth) or cycle of respiration, the device having a conductive stretching material composed of a plurality of filamentous bodies running in the direction of deformation of the sheet material, the filamentous bodies being incorporated into the sheet material in a mutually parallel manner, and the device also having computing means that computes the difference in electrical resistance values from the filamentous bodies that vary in electrical resistance depending on deformation in the in-plane direction of the sheet material, and measuring means that measures movement by the wearer based on the computed results from the computing means.

In clothing using this conductive stretching material, respiration by the person wearing it causes the resistance value to also vary with the size (depth) of respiration, with the cycle fluctuating considerably depending on the speed of respiration. With such clothing, therefore, a fixed time must be ensured as a sampling time for averaging of the resistance values for the conductive stretching material, resulting in a lack of real-time utility. Moreover when cycle fluctuation is added to respiration size it has been impossible to accurately detect resistance values, making it difficult to detect posture at different stages. The invention described was devised in light of this situation and provides a motion measuring device, method and program that can accurately detect posture of the wearer, such as hunching, while excluding the effects of respiration size (depth) or cycle fluctuation, using a simple construction.

The motion measuring device comprises a conductive stretching material having a plurality of filamentous bodies the electrical resistance of which varies with deformation of the filamentous bodies, computing means that computes the difference in electrical resistance from the filamentous bodies of the conductive stretching material, and measuring means that measures movement of the wearer based on the computed results from the computing means. The filamentous bodies of the conductive stretching material are incorporated in the clothing along the direction of deformation of the sheet material and in a mutually parallel manner, each varying in electrical resistance by deformation in the in-plane direction of the sheet material. It is possible to detect different levels of electrical resistance by using different lengths for the filamentous bodies of the conductive stretching material, and having different electrical resistances for the initial states of the filamentous bodies (when not deformed by external force). With this motion measuring device it is possible to accurately detect posture of a wearer, such as hunching, while excluding the effects of respiration size (depth) or cycle fluctuation, by computing the difference in electrical resistance values from pairs of filamentous bodies in the conductive stretching material.

PTL 18 describes a sensor system comprising a plurality of sensors formed of a polymer material having a conductive particle material dispersed at a first dispersion density, a port configured for communication with an electronic module, and a plurality of conductive lead wires formed of the polymer material having the conductive particle material dispersed at a second dispersion density, and connecting between the sensors and the port, wherein each sensor is configured to have increased resistance when deformed under pressure, and the second dispersion density is higher than the first dispersion density, whereby sufficient conductivity is exhibited so that each lead wire is able to transmit an electronic signal between each sensor and the port with the lead wire in any deformed state.

The invention described generally relates to an article of clothing having a sensor system. One embodiment of the invention described relates to a sensor system comprising one or a plurality of sensors formed of a conductive particle material-dispersed polymer material, and conductive lead wires connected to the sensors. The lead wires may also be formed of a conductive particle material-dispersed polymer material. The sensors and lead wires may have the same or different polymer materials and/or conductive particle materials.

According to another embodiment, data transmitted to an external device may be used for one or more purposes. Such purposes may include, most especially, the use of data as control input for a program executed by the external device, such as a game program, or for exercise performance monitoring. Exercise performance monitoring may include monitoring of one or more performance metrics such as, especially, speed, distance, lateral movement, acceleration, jumping height, body weight shifting, ground patterns, balance, foot pronation or eversion, measurement of lifting time during running, lateral cutting force, ground time, center of pressure, throwing arm speed/force, kicking leg speed/force, and body weight distribution and/or impact force.

The sensor described has conductivity (and resistivity) that changes based on deformation and applied force, and may be considered to be a pressure sensitive resistive material. The structure is such that the distances between particles of the conductive material are increased or decreased by deformation of the matrix material, thus changing the resistance or conductivity of the material.

PTL 19 describes a device (of clothing including but not limited to shirts and pants) for detecting and monitoring physiological parameters such as respiration or cardiac parameters. The device described is physiological parameter monitoring clothing having a sensor formed of conductive ink printed on compression clothing, arranged and constructed for robust sensing and a comfortable wear experience. In particular, the clothing described (such as shirts, pants or underwear) is constructed to allow robust sensing of one or more physiological parameters using conductive ink sensors, the conductive ink sensors being directly printed on the clothing, and connected to an interface region of the clothing by conductive tracing (which may or may not be reinforced on the clothing), and the interface regions being connected to an analysis unit such as a microprocessor which is configured to measure, store, process and/or transmit one or more stored parameters.

The conductive ink described is not only conductive but also stretchable, and therefore functions suitably on compression clothing.

Such respiration sensors generally include a strain gauge of a woven fabric and/or conductive ink base. For example, the strain gauge may be formed of the stretchable conductive ink and/or a conductive elastic strip that is described.

PTL 20 describes sensing wear that can simultaneously and successively detect body displacement, such as movement of the extremities, body shape, posture, respiration, chewing, swallowing, pulsation, fetal movement, body potential (electrical heart function) or electrical muscle function, in an essentially noninvasive manner.

A biometric information measuring device is provided that is capable of simultaneously measuring biological displacement and biological potential within a limited area without discomfort, comprising a capacitor type element having a layered structure in the order of, at least, a first stretchable conductor layer 2, a stretchable dielectric layer 3 and a second stretchable conductor layer 4, and a skin contact electrode in which at least the skin contact surface is a stretchable conductor layer, and with biological displacement and biological potential measuring devices provided in an integral manner.

By integrally incorporating a stretchable capacitor that can detect changes in electrostatic capacitance with a skin contact type that contacts with biological skin, the invention described provides a biometric information measuring device that can reduce the area required for sensor mounting and alleviate discomfort for the wearer, while allowing multiple parameters of biometric information to be simultaneously or successively measured.

Since the biometric information measurement device used in the invention described has a high elongation rate in the planar direction it can be suitably used for measurement of the amount of deformation strain in the planar direction, and is able to measure biological displacement and biological potential during walking movement or exercise activity without needing to use gel, paste or adhesive tape between the skin contact electrodes and skin surface.

The stretchable conductor layer of the stretchable capacitor used in the invention described can be obtained by kneading and mixing conductive particles with a flexible resin, and molding it into a film or sheet.

PTL 21 describes vest wear equipped with a motion sensor, the vest wear having an external air side fabric and a skin side fabric, and with a sensor disposed between the external air side fabric and skin side fabric.

The motion sensor which is disposed in a pocket is anchored inside the pocket by a hook- and-loop fastener.

PTL 22 describes a respiration sensor and sensing wear suitable for a stretchable capacitor without hysteresis, exhibiting a 1:1 correspondence between degree of elongation and electrostatic capacitance, and having high sensitivity.

The capacitor that is described has a layered structure with at least a stretchable conductor layer, a stretchable dielectric layer and a stretchable conductor layer stacked in that order, the stretchable conductor layers having a composition comprising metal particles, with a non-elongated resistivity of $3 \times 10^{-3}$ Ωcm or lower and a resistivity at 100% elongation of within 100 times that of the non-elongated resistivity, and a stretchable capacitor is obtained by using the stretchable conductor layers together with the stretchable dielectric layer which has no inorganic components and has a Poisson's ratio of preferably 0.28 or higher. The obtained stretchable capacitor is attached to a belt or shirt to allow sensing of respiration by changes in the vest. It can also read motion by the wearer if it is incorporated into indirect locations of clothing.

PTL 23 describes a device that is able to evaluate joint mobility.

A flexible converter is a conductive elastic body that provides changes in electrical output signals when stretched. The conductive material is a homogeneous mixture of a non-conductive elastic body and conductive particles dispersed in it.

PTL 24 describes a device for monitoring of physiological parameters of the human body.

The monitoring device includes smart clothing, a processing unit and a docking station, the docking station allowing rapid and easy connection/disconnection of a processor. A plurality of knitted fabric sensors are connected to the docking station.

Sensing members disclosed in the prior art have thus been constructed using conductive materials such as conductive fibers or conductive ink, with resistance values changing in response to application of external force, but since the sensor resistance values are low at from several Ω to several 100 Ω, for example, they tend to be affected by contact resistance and have increased power consumption in readout circuits. In addition, such members have low reproducibility for changes in output in response to external force such as tension or load, making it difficult to obtain stable output.

When stretchable wiring is used as conductive material forming the sensing member and a change in electrical resistance value is detected in response to stretching, the sensor responsiveness is determined by the physical properties of the rubber elastomer and the specification of the conductive particles, resulting in poor linearity, difficulty in achieving precise detection of external force due to hysteresis, and poor responsiveness and repeatability when restored to the original state. When a stretchable capacitor is used as a sensing member, the linearity of output with respect to stretching is poor, the responsiveness and repeatability are unsatisfactory, and the weather resistance is inadequate making it unable to withstand prolonged use.

When a piezoelectric material is used as the contact sensing member it is necessary to orient the piezoelectric material or carry out plating for electrode formation, making it difficult to improve productivity, while as of the current time it has also been difficult to produce long processed yarn of 10,000 m or longer. The cost is also is very high, at approximately 1000 to 5000 yen per 1 m of processed yarn. In addition the rigidity of polylactic acid or fluorine resins as piezoelectric materials is also problematic since they cause the piezoelectric thread to have a poor feel, to be difficult to embed into knitted or woven fabrics, and to lack flexibility for use as fiber.

[CITATION LIST]

[PATENT LITERATURE]

[PTL 1] Japanese Patent Publication No. 6025854
[PTL 2] Japanese Patent Publication No. 6689943
[PTL 3] Japanese Unexamined Patent Publication No. 2020-090768
[PTL 4] Japanese Unexamined Patent Publication No. 2020-036027
[PTL 5] Japanese Patent Publication No. 6107069
[PTL 6] Japanese Patent Publication No. 5754946
[PTL 7] Japanese Unexamined Patent Publication No. 2006-234716
[PTL 8] Japanese Unexamined Patent Publication No. 2016-173685
[PTL 9] Japanese Unexamined Patent Publication HEI No. 10-25635
[PTL 10] Japanese Unexamined Patent Publication No. 2013-231246
[PTL 11] Japanese Patent Public Inspection No. 2018-507081
[PTL 12] Japanese Unexamined Patent Publication No. 2011-98214
[PTL 13] Japanese Patent Publication No. 6960725
[PTL 14] Japanese Patent Publication No. 698667
[PTL 15] Japanese Unexamined Patent Publication No. 2018-21270
[PTL 16] Japanese Unexamined Patent Publication No. 2014-25180
[PTL 17] Japanese Unexamined Patent Publication No. 2017-123911
[PTL 18] Japanese Patent Public Inspection No. 2016-509635
[PTL 19] Japanese Patent Public Inspection No. 2017-512542
[PTL 20] Japanese Unexamined Patent Publication No. 2019-72048
[PTL 21] Japanese Unexamined Patent Publication No. 2022-55855
[PTL 22] International Patent Publication No. WO2018/056062
[PTL 23] US Patent No. 4444205
[PTL 24] U.S. Patent Application Publication No. 2016/0113581

SUMMARY

[TECHNICAL PROBLEM]

In light of the technical level described above, the problem to be solved by the invention is to provide a biometric information measurement device comprising a fiber base material with a sensing member, which can be processed in long pieces, has excellent mass productivity, allows use as warp thread for a woven fabric or warp knit, is pliable with an excellent feel, and is produced at much lower cost than contact with a contact sensing fiber member using a conventional piezoelectric material (piezoelectric yarn), while also having lower specific load elongation than a conventional sensing member, which results in satisfactory linearity, virtually no hysteresis, rapid responsiveness when restored to its original state and satisfactory repeatability, and wherein the device is disposed in contact with a predetermined position of the body.

[SOLUTION TO PROBLEM]

As a result of diligent experimentation with the aim of solving this problem, the present inventors have found, unexpectedly, that the problem can be solved by the construction described below, and the invention has been completed upon this finding.

Specifically, the present invention is as follows.

[1] A biometric information measurement device which comprises a fiber base material that includes a sensing member and which is disposed in contact with a predetermined position of the body, wherein the sensing member has at least two core-sheath structure yarns comprising high resistance fiber arranged as covering material around a linear conductor as the core material, two of which are disposed in mutual proximity, and is able to read off the change in resistance and/or change in capacitance between linear conductors of two core-sheath structured yarns, and based on that change, measures biometric information or fluctuation in the same.
[2] The biometric information measurement device according to [1] above, wherein the sensing member is plied structural yarn having two core-sheath structure yarns further twisted together.
[3] The biometric information measurement device according to [1] or [2] above, wherein the high resistance fiber arranged in the sensing member is made of fiber having a carbon-based conductive material provided on at least part of the surface, and is able to read off change in resistance between the linear conductors.
[4] The biometric information measurement device according to any one of [1] to [3] above, wherein the specific load elongation of the sensing member is 5% or lower.
[5] The biometric information measurement device according to any one of [1] to [4] above, wherein the biometric information is change in body posture.
[6] The biometric information measurement device according to any one of [1] to [4] above, wherein the biometric information is related to respiration.

**[7]** The biometric information measurement device according to any one of [1] to [4] above, wherein the biometric information is change in moisture content of a biological surface.

**[8]** The biometric information measurement device according to [3] above, wherein the change in output of a sensor with respect to change in moisture content at the section where the sensing member measures biometric information is 1/20 or lower with respect to change in sensor output for biometric information measurement.

**[9]** The biometric information measurement device according to any one of [1] to [8] above, wherein at least one of the core-sheath structure yarns is a core-sheath structure yarn wherein high resistance fiber as a covering material is wrapped in one direction around a linear conductor as the core material, covering it.

**[10]** The biometric information measurement device according to any one of [1] to [8] above, wherein at least one of the core-sheath structure yarns is a core spun yarn wherein high resistance staple fiber as a covering material is wrapped in random directions around a linear conductor as the core material, covering it.

**[11]** The biometric information measurement device according to **[1]** above, wherein two or more of the core-sheath structure yarns are disposed in partial contact inside the fiber base material.

**[12]** Clothing in which a biometric information measurement device according to any one **of [1] to [11]** above is disposed.

**[13]** Clothing in which a biometric information measurement device according to [11] above is disposed.

[ADVANTAGEOUS EFFECTS OF INVENTION]

A sensing member used in the biometric information measurement device of the invention can be processed to long lengths, has excellent mass productivity, allows use as warp yarn in woven fabrics or warp knitting and is pliable with an excellent feel, while it can also be provided at much lower cost than contact sensing fiber members (piezoelectric threads) for contact using conventional piezoelectric materials. Specifically, since the sensing member allows sensing of a load or tensile force using the common fiber materials polyester or nylon, it is possible to obtain contact sensing fibers at very low cost, and since it includes core spun yarn and employs established covering technology for processing of the fibers, it is possible to achieve processing to long lengths and to obtain excellent mass productivity, while also obtaining processed yarn with a highly superior feel compared to piezoelectric yarn, thereby facilitating formation into fiber members such as woven or knitted fabrics.

The sensing member used in the biometric information measurement device of the invention varies in electrostatic capacitance and/or resistance value, allowing detection of the state of continuous application of a load or tensile force.

With the sensing member used in the biometric information measurement device of the invention, if the core-sheath structure yarn having high resistance staple fiber as a covering material wrapped in random directions around a linear conductor as the core material to cover it (also commonly referred to as "covering yarn") is core spun yarn, then it will be easier to use natural fiber and biodegradable yarn, and easier to impart functionality to the sheath yarn.

Sensing members of the prior art utilize sensor output such as electrical resistance and electrostatic capacitance in response to elongation of stretchable wiring, which depends primarily on the physical properties of the rubber elastomer, while exhibiting poor linearity with hysteresis as well as slow responsiveness when restored to the original state, and consequently the repeatability has been less than satisfactory. In contrast, the sensing member used in the biometric information measurement device of the invention has lower specific load elongation than a conventional sensing member, and therefore exhibits satisfactory linearity, virtually no hysteresis, fast responsiveness when restored to the original state, and satisfactory repeatability.

The biometric information measurement device of the invention can therefore make use of wear (such as a T-shirt, underwear, vest or belt) containing a sensing fiber member (yarn) having plied structural yarn in which two of the core-sheath structure yarns are further twisted together, to detect displacement or movement such as slight elongation in the body surface, body pressure such as foot or finger pressure, or fluctuation in moisture content. This allows detection of respiration, posture changes, movement of the wrists, ankles or fingers, movement of muscles (such as pelvic floor muscles, brachioradialis muscles or calves), wear pressure by compression wear or socks, foot sole pressure or foot pressure distribution, swallowing motion, analysis of walking or running motion (for rehabilitation purposes or preventive care), or fluctuation in moisture content due to sweating or excretion. In particular, the biometric information measurement device of the invention allows simultaneous detection of the state of respiration and quality of posture, and also detection of multiple signals for simultaneous detection of opening and closing of the hand (fist and open palm) or bending of the wrist.

BRIEF DESCRIPTION OF DRAWINGS

Fig. 1 is a schematic diagram of a conventional piezoelectric processed yarn.
Fig. 2 is a schematic diagram showing one embodiment of a sensing member.

Fig. 3 shows the outer appearance of a sensing member which is plied structural yarn, and a magnified photograph of the same.

Fig. 4 is a photograph of a narrow woven fabric with interweaving of a sensing member as plied structural yarn.

Fig. 5 is a photograph of a sensing member in the form of a plain weave woven fabric where the warp yarn and weft yarn are each core-sheath structure yarn covered by wrapping high resistance fiber as a covering material in one direction around a linear conductor as the core material.

Fig. 6 is a schematic diagram showing a device system for measurement of change in resistance between two core-sheath structure yarns covered by wrapping high resistance fiber as a covering material in one direction around a linear conductor as the core material.

Fig. 7 is a graph showing an example of output current value (sensor output) during application of a load, for a sensing member of the embodiment, as plied structural yarn.

Fig. 8 is a graph showing the relationship between rate of change in applied load and current value (sensor output) for a sensing member of the embodiment, as plied structural yarn.

Fig. 9 is a graph showing an example of output current value (sensor output) during application of a load or tensile force with a sensing member in the form of a plain weave fabric (narrow woven fabric), where the warp yarn and weft yarn are each core-sheath structure yarn covered by wrapping high resistance fiber as a covering material in one direction around a linear conductor as the core material.

Fig. 10 is a schematic diagram of a production apparatus for core-sheath structure yarn.

Fig. 11 is a schematic diagram of core-sheath structure yarn obtained by the production apparatus.

Fig. 12 is an illustration of the principle of measurement of the electrical characteristic between a pair of core-sheath structure yarns.

Fig. 13 is a schematic diagram of a conventional piezoelectric processed yarn.

Fig. 14 shows the outer appearance of a sensing member which is plied structural yarn using staple fiber, and a magnified photograph of the same.

Fig. 15 is a graph showing an example of output current value (sensor output) for a sensing member as plied structural yarn using staple fiber.

Fig. 16 is an illustration for Example 1.

Fig. 17 is a graph showing the results of sensing in Example 1.

Fig. 18 is an illustration for Example 2.

Fig. 19 is a graph showing the results of sensing in Example 2.

Fig. 20 is an illustration for Example 3.

Fig. 21 is a graph showing the results of sensing in Example 3.

Fig. 22 is an illustration for Example 4.

Fig. 23 is a graph showing the results of sensing in Example 4.

Fig. 24 is an illustration showing a woven state of sensing yarn formed by smooth weaving at the center section of the wristband in Example 4.

DESCRIPTION OF EMBODIMENTS

An embodiment of the invention will now be described in detail.

The first embodiment of the invention is a biometric information measurement device which comprises a fiber base material that includes a sensing member and which is disposed in contact with a predetermined position of the body, wherein the sensing member has at least two core-sheath structure yarns comprising high resistance fiber arranged as covering material around a linear conductor as the core material, two of which are disposed in mutual proximity, and is able to read off the change in resistance and/or change in capacitance between linear conductors of two core-sheath structured yarns, and based on that change, measures biometric information or fluctuation in the same.

As used herein, the term "biometric information" refers to changes in movement or posture, changes in position due to respiration, for example, body pressure, or changes in moisture content on the biological surface, of a human or animal body. The biometric information measurement device of the invention allows highly precise measurement of such biometric information by a convenient method.

The biometric information measurement device of the embodiment may be provided in an appropriate form depending on the type of biometric information to be measured. For measurement of respiration, for example, since movement of the body caused by respiration depends on motion of the lungs, the fiber base material is fitted to the body so that sensing members are positioned in at least one location from among the chest region near the diaphragm corresponding to the 4th to 5th ribs from the bottom, and the abdominal region, neck and chest region at the upper part of the lungs.

Alternatively, for measurement of body posture, in order to detect a "hunched state", it is preferred to detect the lateral length of the back side of the body, especially between the scapula, and therefore the fiber base material preferably has the sensing member mounted in the lateral direction on the back side. For detection of movement of hand opening or fist clenching as biometric information, both of which involve greater movement of the forearm muscles, the fiber base material has the sensing member disposed in the brachioradialis muscle or wrist area, for example. For measurement of finger movement, the fiber base material may have sensing members disposed parallel to the lengthwise directions of the fingers.

The form of the fiber base material may be as a woven fabric or a knitted fabric, with no limitation to the structure of the fiber base material. A nonwoven fabric or paper sheet may also be used instead. Such forms may be any of various types of wear such as underwear, T-shirts, work clothes or vests, or wristbands, supporters, gloves, finger cots, or belts, selected as appropriate for the metric to be measured. For measurement of movement of the body as a whole, such as respiration or posture, the wear is preferably a shirt or tank top that fits comfortably onto the body, from the viewpoint of allowing both a satisfactory feel during wear and precise measurement to be achieved in a simple manner.

The number of sensing members used may be a single one at one location where the body movement to be measured is greatest, or it may be more than one at multiple locations. For measurement of respiration, for example, measuring movement at multiple locations including the chest, abdominal region, and neck is preferred to not only allow measurement of simply the respiration rate, but also to obtain information relating to the quality of respiration, including distinguishing between abdominal breathing and thoracic breathing, detecting shallow breathing, and determining if respiration involves the entire body. In the case of deep breathing with large movement of the diaphragm, for example, large output fluctuation is obtained by both sensing members disposed at the diaphragm and the abdominal region, whereas shallow breathing can be judged if change in the abdominal region is relatively low.

Two or more sensing members may also be fitted at the target measuring site. For example, in order to measure respiration, two or more sensing members may be inserted near the diaphragm and the sum or difference in their output may be computed to increase the precision, and reliability, and avoid missing measurement. Reading the sum of outputs from multiple sensing members fitted at adjacent sites allows greater output to be obtained more conveniently, providing an advantage by significantly lowering the chance of missed measurement. When the difference of the outputs is to be taken, it is possible to eliminate variation in output not related to respiration, such as the effects of noise or disturbance around the body, to allow more precise measurement to be performed.

The output of each sensing member is read out using a readout circuit electrically connected to the sensing member. The readout circuit used may comprise, for example, a current/voltage conversion circuit, an analog/digital conversion circuit, a filter circuit, an amplifier and an interface unit. It may further comprise a communication unit if necessary, for readout of the sensing member output to an external device by wireless communication. In the case of wired communication, it may be electrically connected directly to an exterior signal device via an interface unit. Output from the sensing member may be via wireless communication or wired communication, but using wireless communication such as Bluetooth[R], BLE (Bluetooth[R] Low Energy), Zigbee, WiFi, NFC or LPWA is more preferable from the viewpoint of allowing measurement of biometric information while allowing free movement of the body. The use of wired communication is preferred from the viewpoint of more precise communication of accurate measurement with less leakage. Computing the outputs from multiple sensing members may be carried out on a readout circuit, or it may be carried out in an external device after the output has been transmitted.

One embodiment of sensing member to be used in the biometric information measurement device of the embodiment is a sensing member having at least two core-sheath structure yarns covered by wrapping a high resistance fiber as a covering material in one direction around a linear conductor as the core material, two of which are disposed in mutual proximity, wherein change in resistance and/or change in capacitance (i.e. change in impedance) between the linear conductors of the two mutually adjacently disposed covering yarns is read off.

Another embodiment of the sensing member is a sensing member having at least two core-sheath structure yarns comprising high resistance fiber arranged as covering material around a linear conductor as the core material, two of which are disposed in mutual proximity, wherein at least one of the core-sheath structure yarns is core spun yarn covered by wrapping high resistance staple fibers as covering materials in random directions around a linear conductor as the core material, and change in resistance and/or change in capacitance between the linear conductors of the two mutually adjacently disposed covering yarns is read off.

The linear conductor as the core material (core yarn) is not particularly restricted so long as it is electroconductive, and it may be a linear conductor in which the material itself is conductive, for example, conductive fibers such as carbon fibers or metal fibers, or it may be a linear conductor in which non-conductive fibers have been imparted with electroconductivity. In the former case, carbon fibers prepared as fiberized carbon are preferred for high durability in moisture sensing, as described below. A fiberized SUS material is preferred as it can ensure rust resistance and simplify end processing including connection with circuits. In the latter case, using a metal plating such as silver or copper around a filament of nylon or the like, or a metal foil wrapped around a tape-like filament, or an aerosol-like electric conductor adhered onto a fiber surface by spraying, is preferred from the viewpoint of improving the feel and flexibility. In this case, the conductive fibers are preferably composed of multifilaments to obtain satisfactory conductivity and increased strength. Using high-strength fibers such as polyallylate or aramid instead of nylon can further increase the tensile strength. Alternatively, the linear conductor used may be one imparted with conductivity by using stretchable metal ink around an elastic solid such as urethane or silicone. This will allow a stretchable fiber member to be obtained. The linear conductor used may also be a linearized mixture of a conductive material and an insulating material. For example, using a material obtained by linear processing of a material mixture of a carbon-based conductive material or a metal mixed with a resin such as nylon or polyester can yield a significantly lower-cost linear conductor, though with some reduction in conductivity. A linear conductor may also be one or more metal wires, from the viewpoint of lower cost even while exhibiting a poorer feel. For example, the strength can be drastically increased by using metal wire with a diameter of about 30 $\mu$m to 1 mm.

The linear conductor has a conductive fiber fineness of preferably 10 dtex to 15,000 dtex and more preferably 20 dtex to 5000 dtex, from the viewpoint of tending to provide a more satisfactory feel. In the case of a multifilament, the single fiber fineness is preferably 1 dtex to 30 dtex and more preferably 2 dtex to 10 dtex, from the viewpoint of tending to provide a satisfactory feel and high conductivity. The number of filaments is more preferably 10 to 200. The number of filaments is preferably 10 or more from the viewpoint of more easily obtaining a satisfactory feel and ensuring satisfactory conductivity. An excessively large number of filaments, however, may increase cost and may also further increase the rigidity, thus impairing the feel. It is for these reasons that the aforementioned range for the number of filaments is preferred.

The conductive material forming the linear conductor may be the same material for both core-sheath structure yarns of a pair, or they may be of different materials, using any desired combination of materials. For sensing of contact, load or tension, it is preferred to use the same conductive material to allow more efficient production. For sensing of liquids such as moisture, using different materials for the linear conductors in pairs of two core-sheath structure yarns causes generation of voltage or current by an electrochemical effect known as galvanic action when the liquid forms an adhering bridge between the two different materials, thus allowing sensing of liquids without a power source. Examples of combinations of different materials include combinations of iron and copper, iron and silver, aluminum and copper, and silver and copper.

As used herein, the term "high resistance fiber" for the covering material (also "cover yarn" or "sheath yarn") includes the concept of fiber that can provide electrical insulation between any pair of two linear conductors as a core materials, as well as insulating fiber having an innate insulating property, piezoelectric materials such as polylactic acid (PLA), and ferroelectric substances such as polyvinylidene fluoride (PVDF), without restriction so long as they can form high resistance or capacitance between the two conductors. However, in order for the covering material (cover yarn) to be able to cover the linear conductor as the core material while being stationary without forming a gap, and preventing electrical shorting, it preferably includes either multifilament high resistance fiber or high resistance spun yarn (staple fiber) that can produce evenness and a homogeneous covering thickness, from the viewpoint of the coverage, sensing performance and feel, and most preferably it consists of multifilament high resistance fiber or high resistance spun yarn. The high resistance fiber material is not particularly restricted so long as insulation of a certain level is ensured when in a state without sensing action such as contact, tension or liquid contact (an idling state), but from the viewpoint of cost and availability, polyester (PE), nylon (Ny, polyamide), epoxy or acrylic synthetic fibers are preferred, while natural fibers such as cellulose fiber, semisynthetic fibers or regenerated fibers such as cupra may also be used. High resistance fiber materials to be used include piezoelectric elements or ferroelectric substances, such as polylactic acid (PLA) or polyvinylidene fluoride (PVDF), and biodegradable resins. A piezoelectric element can ensure insulation in an idling state and produce an output signal corresponding to the piezoelectric characteristic during application of stress, thus increasing the sensor sensitivity. From the viewpoint of the cost and feel of formed woven or knitted fabric, however, it is preferred to use fibers commonly used in clothing, such as polyester, nylon or acrylic fibers.

For sensing of changes in resistance, a material that imparts slight conductivity to the insulating fiber may be used as the cover yarn (sheath yarn) of high resistance fiber. The range of conductivity for the sheath yarn may be a range in which change in resistance between linear conductors of two mutually adjacently disposed core-sheath structure yarns can be read off. Specifically, the value of the resistance (sensor resistance) between linear conductors of mutually adjacently disposed core-sheath structure yarns is preferably in the range of 0.5 k$\Omega$ to 5 G$\Omega$, and the sensor resistance value is preferably 20 times to $1 \times 10^9$ times the value of the resistance of the linear conductors alone (wiring resistance). This will sufficiently increase the sensor resistance value with respect to the wiring resistance value without causing electrical shorting, when voltage is applied between two linear conductors forming two mutually adjacent core-sheath structure yarns, thus allowing load and tensile force to be accurately detected without being affected by the wiring resistance. The sensor resistance value is more preferably in the range of 0.5 k$\Omega$ to 100 M$\Omega$ from the viewpoint of simplicity of the readout circuit. The range for the electric resistivity of the sheath yarn material is preferably $10^4$ $\Omega\cdot$m to $5 \times 10^9$ $\Omega\cdot$m from the viewpoint of easily satisfying the aforementioned sensor resistance range.

The material of the sheath yarn that has imparted slight conductivity may be a material comprising a conductivity-imparting material, such as a carbon-based conductive material, metal particles, a metal sulfide such as copper sulfide or a metal oxide such as tin oxide or zinc oxide, added to a polyester, nylon or acrylic insulating material. Alternatively, the sheath yarn used may be a mixture of both insulating fiber and conductive fiber. For example, carbon-based fibers having carbon present on at least portions of the surfaces, such as KURACARBO (registered trademark of Kuraray Co., Ltd.) or Beltron (registered trademark of KB Seiren, Ltd.), which are commercially available as antistatic fibers, or fibers plated with metal compounds, such as THUNDERON (registered trademark of Nihon Sanmo Dyeing Co., Ltd.), may be selected to obtain the desired sensor resistance value.

Using core spun yarn (CSY) (mentioned below) facilitates the use of natural fibers or biodegradable fibers that are difficult to use as long fibers, so that their respective properties can be exhibited.

The sensing member preferably uses a combination of quick-drying fibers as the material for the covering material (cover yarn). For sensing of moisture or ethanol, in particular, using quick-drying fibers for the cover yarn allows drying in a short period after contact with moisture and the like, allowing the original state to be restored more quickly. Quick-drying fibers may be synthetic fibers with a low moisture content, but in order to exhibit both water absorption and quick-drying performance they are most preferably a combination of synthetic fibers with cellulose fibers. Synthetic fibers in this case are preferably polyester, nylon or acrylic fibers, cellulose fibers are preferably natural cellulose fibers such as cotton or hemp, regenerated cellulose fibers such as rayon, polynosic, lyocell, cupra or modal fibers, or semisynthetic fibers such as acetate, with multifilament long fibers being especially preferred. A combination of both fibers may be a blend of both fibers for cover yarn, or in the case of double covering, it may be covering with synthetic fibers and cellulose fibers.

The fineness of multifilament high resistance fibers used as the covering material (cover yarn) is preferably 15 dtex to 25,000 dtex and more preferably 30 dtex to 8000 dtex, from the viewpoint of helping to prevent electrical shorting between pairs of linear conductors. The single fiber fineness of a multifilament high resistance fiber is preferably 1 dtex to 10 dtex and more preferably 2 dtex to 8 dtex, from the viewpoint of more easily obtaining a satisfactory feel.

The fineness of high resistance spun yarn used as the covering material (cover yarn) is preferably a thread size of 60 to a thread size of 5, and more preferably a thread size of 30 to a thread size of 10, from the viewpoint of helping to ensure insulation.

There are also no particular restrictions on the method of producing core-sheath structure yarn having high resistance fiber as a covering material around a linear conductor as the core material, which is arranged in the sensing member of the embodiment, but the following method described in PTL 9 may be mentioned as an example.

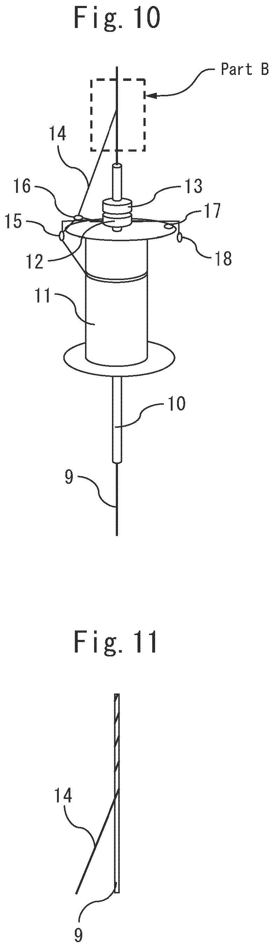

Fig. 10 is a schematic diagram where a bobbin wound with cover yarn (covering material) (14) is loaded in while operating a covering device equipped with a double-drive flyer (12). Fig. 11 shows a magnified view of section B in Fig. 10. The core yarn (core material) 9 passes through the hollow section of a hollow spindle 10, and then through a snail guide above it (not shown), finally being taken up onto a take-up roll (not shown). The cover yarn 14 is passed through one drive guide 15 and 16 of the double-drive flyer 12 and reeled from the bobbin by rotation of the hollow spindle (in tandem with the bobbin), and the cover yarn 14 is passed through the snail guide while being wound around the core yarn 9, and is taken up. The reason for having two drives of the flyer 12 is in order to provide balance to the flyer 12 when the flyer rotates.

Double covering may also be carried out, whereby covering devices are arranged vertically in two levels and two different cover yarns (either of the same type or different types) from two bobbins are used for covering in order. Most preferably, each cover yarn is used for covering in the same direction (the two cover yarns being both S-twisted or Z-twisted), so that the thickness can be made uniform and the gaps between the insulating fibers can be reliably filled, allowing the sensing performance to be improved.

The cover yarn may also be false twisted finished yarn (woolly yarn), from the viewpoint of more easily improving the feel and coverage.

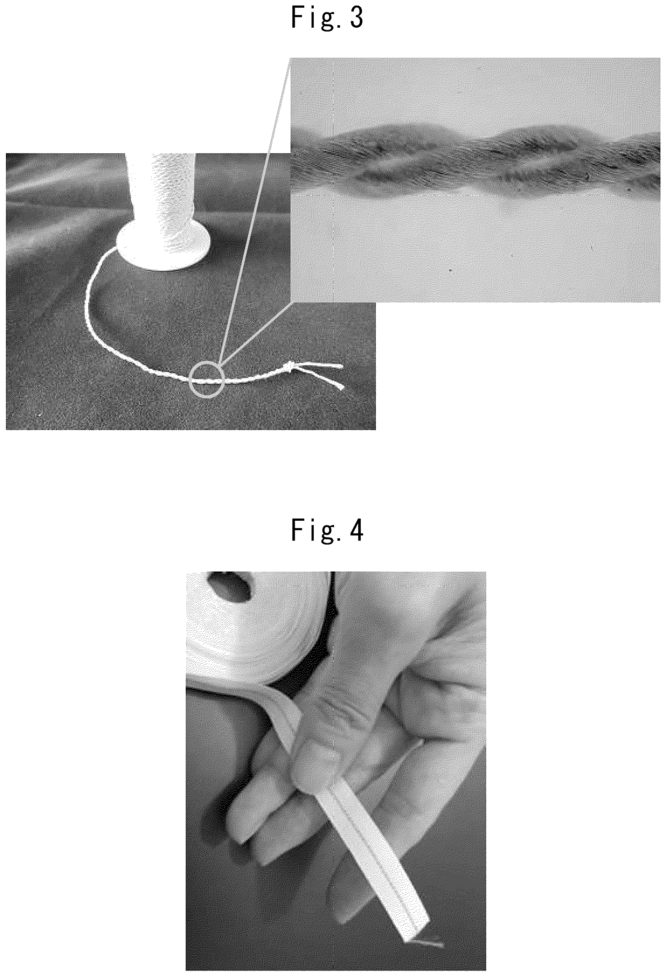

Fig. 2 and Fig. 3 show examples of two core-sheath structure yarns (7) that are further twisted together, as a mode of the sensing member of the embodiment. According to the invention, yarn with this structure is referred to as plied structural yarn (8) or plied yarn. In plied structural yarn, the relationship between the wrapping direction of the covering material (cover yarn) in each core-sheath structure yarn and the twisting direction when two core-sheath structure yarns are further twisted together is not particularly restricted, but preferably the wrapping directions of the covering materials (cover yarns) (6) disposed around the linear conductor as the core material (5) are the same for two mutually adjacent core-sheath structure yarns (7), and preferably the two core-sheath structure yarns are plied structural yarns twisted together in the direction opposite from the wrapping direction of the covered yarn (cover yarn). If the plied structural yarn is twisted in the direction opposite from the wrapping direction of the covering, the torque of the final yarn will be weakened and handling will be facilitated during the production steps. Plied structural yarn naturally has the two core-sheath structure yarns situated mutually adjacent, with the two core-sheath structure yarns having crossing contact points.

The twist coefficient K of the core-sheath structure yarn, represented by the following formula:

$$\text{Twist coefficient K} = (\text{SS} + \text{SC})^{1/2} \times \text{R}$$

{where SS is the fineness (dtex) of the linear conductor as the core material, SC is the total fineness (dtex) of the covering material, and R is the wrapping number (number of twists) of the covering material (twists/m)}

is preferably 7000 to 30,000. A twist coefficient K of 7000 or greater will tend to prevent electrical short circuiting between the two linear conductors, while 30,000 or lower will help to produce greater sensor output. In the case of double covering, the twist coefficient is calculated during covering of the first and second layers, and the average value is used.

The core-sheath structure yarn of the sensing member according to another mode has a structure in which high resistance staple fiber as a covering material around a linear conductor as the core material, covers by being wrapped in random directions. It is preferably core spun yarn (CSY) covered by inserting the core yarn during spinning of staple fiber as the sheath yarn.

The present inventors have examined the use of core spun yarn as core-sheath structure yarn, and have found that sensing performance can be exhibited by ensuring the insulating property for the core yarn. However, it was also found that the insulating property can be partially impaired resulting in lower sensing performance, due to variation in the state of covering under ordinary conditions, and core spun yarn exhibiting continuous excellent sensing performance was successfully produced by establishing specific covering conditions.

Specifically, it is important to set a specified range for the weight proportion between the core yarn and sheath yarn. Ordinary core spun yarn has a core yarn mixing ratio of 15 mass% to 30 wt%, but according to the invention the core yarn mixing ratio is preferably 5 mass% to 12 wt% and most preferably 10 wt% or lower.

For this embodiment, at least one from among two or more core-sheath structure yarns must be core-sheath structure yarn comprising high resistance staple fiber as a covering material wrapped in random directions around a linear conductor as the core material, covering it, where the core-sheath structure yarn is preferably core spun yarn.

The type of core material and the fineness may be the same as the core-sheath structure yarn described above, and the single fiber fineness of the high resistance staple fibers wrapped random as the covering material (cover material) is preferably a thread size of 30 to a thread size of 5, and more preferably a thread size of 20 to a thread size of 5, from the viewpoint of helping to ensure insulation. The degree of wrapping may be adjusted so that the core yarn mixing ratio is within the aforementioned preferred range.

As shown in the example in Fig. 4, the sensing member may be in the form of a narrow woven fabric with the plied structural yarn running continuously in one direction of the woven fabric. In the example of Fig. 4 the plied structural yarn is woven as warp yarn at the widthwise center section of the narrow woven fabric, but the plied yarn may be used as either or both the warp yarn and weft yarn, in any number depending on the number of locations where sensing is desired. From the viewpoint of continuous production, it is preferred for the plied structural yarn to be used as part of the warp yarn. This will allow detection of contact or load from objects at the sections where the plied structural yarn has been interwoven, and/or sensing of changes in liquid contact or changes in humidity. When the plied structural yarn has been interwoven into part of a narrow woven fabric, the shape of the fiber member adopts a tape-like form, which is advantageous for fiber products such as clothing or bags as compared to using the plied structural yarn alone. The width of the woven fabric containing the plied structural yarn is preferably 1 to 200 mm and more preferably 5 to 30 mm. The use of fibers other than the plied structural yarn is not particularly restricted, and the woven texture is also not particularly restricted. In order to help prevent static electricity in a fiber base material comprising a fiber sensing member of the embodiment, the plied structural yarn may be wrapped with antistatic thread interwoven with a conductive material, with the yarn being embedded in the woven fabric. The antistatic thread used may have an electrical resistance value of $10^6$ to $10^{10}$ $\Omega$/cm per unit length, and for example, it may be "BELTRON$^R$" carbon, Beltron type or white Beltron type by KB Seiren, Ltd., or "KURACARBO$^R$" by Kuraray Co., Ltd. In a fiber base material comprising a fiber sensing member of the embodiment, a similar effect can also be obtained by arranging antistatic thread near the member or between multiple disposed members.

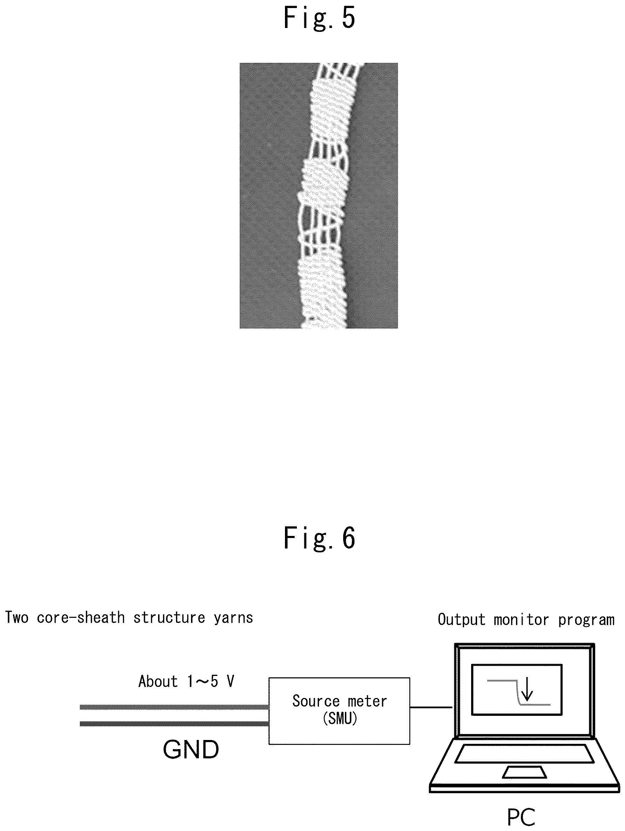

A woven fabric having multiple plied structural yarns for warp and weft may also be used (see Fig. 5). In Fig. 5, five rows of warp yarn are arranged with weft yarn woven at left and right, but the braided woven fabric form is not limited to such a structure. Using such a sensing member with multiple interwoven plied structural yarns allows simultaneous measurement of applied external forces such as loads or tensile forces at the locations of each of the plied structural yarns, so that the locations of applied external forces can be mapped.

Alternatively, the core-sheath structure yarn may be used as both warp yarn and weft yarn in a woven fabric. In this case, two core-sheath structure yarns are disposed adjacently at intersections between warp and weft thread, to exhibit the aforementioned sensing function at those sections for application as a sensing fiber member. The form of the woven fabric may be any desired form.

The sensing member of the invention provided in such a fabric form differs from conventional elongation sensors with stretchable wiring in that it exhibits low extensibility (resistance to elongation contraction), satisfactory linearity, virtually no hysteresis, and rapid responsiveness when restored to the original state so that it has satisfactory repeatability. Specifically, it is characterized by a low elongation rate (specific load elongation) of preferably 5% or lower and more preferably 3% or lower, under repeated load of 1 N on a 1 cm-wide sample (described below). The lower limit is not particularly restricted but is preferably 0.1% or higher and more preferably 0.5% or higher in order to ensure that biometric information is properly tracked.

The plied structural yarn can also be arranged in a knitted fabric. Alternatively, two or more core-sheath structure yarns may be arranged in a partially adjacent or crossing manner to exhibit a sensing function.

A fiber base material with arranged sensing members may be in an appropriate extensible form depending on the purpose. For example, by using plied structural yarn as part of the knitting yarn forming a knitted fabric, it is possible to provide an elongatable portion of 20% or greater or 50% or greater of the fiber base material when the sensing member is knitted into the fiber base material.

The core-sheath structure yarn may also be used as top thread or bottom thread of a sewing machine during sewing or embroidery of the fiber base material. This will provide one core-sheath structure yarn each above and below the fiber base material, to exhibit the aforementioned sensing function at the contacting points of the core-sheath structure yarn for application as a sensing member.

When producing a fiber base material such as a woven or knitted fabric, the linear conductor in each pair of two core-sheath structure yarns more preferably has one of the electrode leads (parts for mounting onto a circuit) leading out on the front side of the fiber base material and the other on the back side of the fiber base material. Most preferably, all of the linear conductors used for voltage application lead out on the same side of the fiber base material, with the signal output linear conductors leading out on the opposite side. This preferred example is advantageous in that when a plurality of core-sheath structure yarn linear conductors are electrically connected and voltage is applied, and each of the signals is independently read off, it is possible to prevent electrical short circuiting while occupying less space, and also in that mounting is simplified, thus improving productivity.

Fig. 6 is an overview diagram showing a device system for measurement of change in resistance between two core-sheath structure yarns covered by wrapping a multifilament high resistance fiber as a covering material in one direction around a linear conductor as the core material, but there is no particular limitation to this device system. The conductive fibers are open at the ends of the pair of two core-sheath structure yarns, and a source meter (SMU: source major unit) is connected allowing simultaneous measurement of voltage, current and resistance upon supply of voltage and current, allowing measurement of the resistance between the pair of core-sheath structure yarns. Instead of using this type of measuring instrument, a readout circuit may be formed using an analog/digital conversion circuit, current-voltage conversion circuit or amplification circuit, for use in measuring the resistance.

The principle of sensing here is based on change in impedance between linear conductors as the form of the core yarn or sheath yarn changes under application of a load or tension. Several principles may be mentioned for change in impedance which simultaneously produce different phenomena, and therefore the specific mechanism is not limited. One of the principles for change in impedance is a change in distance between core yarns or electrode width between opposing core yarns due to application of external force, whereby the electrostatic capacitance or resistance value between the core yarns changes. Another possible principle is that the threads composing the sheath yarn adhere together more closely due to shape deformation of the sheath yarn under application of external force, thus lowering the resistance value as numerous conductive paths are formed.

In the example of the embodiment, for example, when using an insulator on a sensing section area of 8.75 $mm^2$, application of an approximately 3 N load (corresponding to a pressure of $3.43 \times 10^5$ Pa) produces conversion to R of between 3.5 GΩ and 1.5 GΩ, with a very large change for $\Delta R/R$ of -57%. The electrostatic capacitance C in this case varies from 3.31 pF to 3.53 pF, with a $\Delta C/C$ of 6.7%. With such a large change in resistance value, the sheath yarns come into closer contact, resulting in more contact points and increasing the microcurrent flowing through the sheath yarns, in addition to change in impedance due to simple shape deformation between the core yarns. For insulating fibers forming sheath yarns, absolutely no current flows between the two linear conductors if an insulator is an ideal insulator, but an actual practical insulator is slightly conductive, with a known electric resistivity of $10^6$ to $10^9$ Ω·m. The mechanism of electrical conduction in an insulating polymer is known to be hopping conduction, whereby electrons and ions traverse through local states. Although ideally no charged particles are present in an insulating polymer, actual polymer materials contain impurities such as catalysts and moisture introduced during the manufacturing process, and the dissociated ions resulting from such impurities react to the applied electric field and move, generating a weak current. According to one mode of the invention, a conductive material such as a carbon material is preferably incorporated into the sheath yarn to produce very slight conductivity, so that it can be read using a simpler circuit. When a sheath yarn with only very little conductivity is placed between two adjacent linear conductors and a load or tensile force is applied to the structure, the fibers composing the sheath yarn come into closer contact with each other, increasing their electrical contact points and causing the value of the weak current to increase. According to this principle, therefore, the sensor resistance decreases, allowing detection of load or tensile force.

The principle of change in impedance may also be based on the theory of change in the space charge limited current with change in distance between linear conductors. As explained below, when objects in contact are conductive, parasitic capacitance is present in addition to changes in electrostatic capacitance, with changes in the parasitic capacitance also being superposed over changes in electrostatic capacitance due to shape deformation.

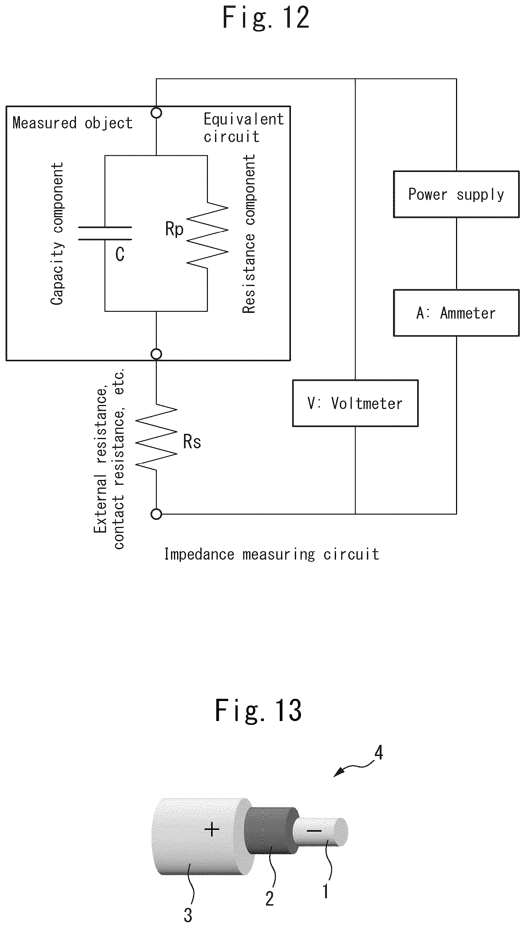

The principle of measurement of electrical characteristics between pairs of core-sheath structure yarns can be considered for an equivalent circuit consisting of a resistor (R) and condenser (C), as shown in Fig. 2 and Fig. 12.

When a sensing signal is to be read off from a change in resistance value, a DC power supply can be used as the electric power supply represented in Fig. 12.

When a change in electrostatic capacitance is to be read off, an AC power supply may be used, applying alternating current with a frequency f between linear conductors in the pair of core-sheath structure yarns and detecting change in impedance. For measurement of capacitance, a common measuring instrument or circuit can be used, such as an LCR meter or an impedance analyzer. Alternatively, a lock-in amplifier circuit can be used in combination, whereby an AC signal is used as a reference signal and the output signal and reference signal are multiplied for frequency analysis. This provides an advantage in that even small changes in electrostatic capacitance can be measured with greater precision. For reading of changes in electrostatic capacitance, on the other hand, a DC power supply as the power source may be used to apply voltage between two linear conductors, reading off the time-dependent change in impedance between them (by monitoring changes in current). Using a DC power supply is advantageous in that it allows measurement to be made using a very inexpensive circuit. The electrostatic capacitance between two electrodes in this case can be described by the following formula:

$$C = \varepsilon(S/L)$$

{where $\varepsilon$ is the dielectric constant between the two electrodes, S is the electrode area, and L is the distance between the electrodes}.

Based on the formula C = $\varepsilon$(S/L), the electrostatic capacitance C is inversely proportional to the distance L between the conductors and proportional to the electrode area. When the object to be detected by contact or the like is an insulator without charge, the distance between the pair of linear conductors decreases and the electrode area undergoes essentially no change, resulting in an increase in electrostatic capacitance, thus allowing detection of contact with the object. When the contacting object is conductive, parasitic capacitance caused by contact with the object is further added, producing a further change in electrostatic capacitance. For example, in the example of the embodiment described above (where $\Delta$R/R is -57%), when a load of about 3 N has been applied using an insulator, C changes from 3.31 pF to 3.53 pF, for $\Delta$C/C of 6.7%, but when a load of about 3 N is applied using a grounded conductive material, C changes from 3.31 pF to 3.01 pF, for a $\Delta$C/C of -8.9%.

When the contacting object is conductive, cumulative parasitic capacitance and increased leakage of charge to ground causes the apparent electrostatic capacitance to decrease. It is thus possible to detect contact or load by reading changes in electrostatic capacitance caused by the contact, load or tensile force. The cover yarn and external air (the external air being normal air when in the atmosphere, or a vacuum when in a vacuum, or replacement gas, when in replacement gas) act as insulators between two linear conductors for this embodiment, with the principle of current flowing between them differing depending on the conditions such as the distance and applied voltage between the electrodes, and the humidity of the external air. Other principles such as leakage current and ionic conduction may also apply in addition to the aforementioned space charge limited current, and either or both changes in resistance value and/or changes in electrostatic capacitance can be read off as output signals.

The sensing member used in the biometric information measurement device of the embodiment can detect changes in impedance between two linear conductors caused by external activity, thus detecting the external activity. For example, a change in distance between the two linear conductors caused by application of contact or load, or a change in the number of electrical contact points between sheath yarns, will result in a change in the resistance value and/or electrostatic capacitance (impedance) between the two linear conductors, thus allowing detection of the contact or load. In cases where the external activity is tensile force or bending stress as well, changes in the distance between linear conductors produce changes in impedance, allowing the external activity to be detected. By using the sensing member, it is possible to obtain sensor output corresponding to slight displacement or motion of stretching on the body surface, or to respiration, posture changes, movement of the wrists, ankles or fingers, movement of muscles (such as pelvic floor muscles, brachioradialis muscles or calves), wear pressure by compression wear or socks, foot sole pressure or foot pressure distribution, swallowing motion, or walking or running motion.

Alternatively, when a substance that can change the impedance between two linear conductors is added, the presence or absence of that substance can be detected. For example, when non-ultrapure water such as tap water, or brine, an ion beverage or a water/ethanol mixture is dropped between two linear conductors, the resistance value between the linear conductors decreases significantly, increasing the current value between them and thus allowing detection of the presence or absence of the liquid. Changes in humidity likewise cause changes in impedance, allowing use as a humidity sensor.

For detection of fluids with very slight conductivity, such as tap water or human body sweat, the cover yarn material used is a material with lower conductivity than the conductivity of the fluid to be detected. This allows detection of fluids by changes in impedance of the sensor before and after infiltration of the fluids. For example, when carbon-based fiber is used for the cover yarn, since the sensor resistance value is somewhat low and carbon-based fiber conducts current more easily than moisture, detection of moisture is not possible.

Alternatively, application of contact or external force such as load or tensile force, and contact with a fluid such as moisture, can be detected simultaneously. Since the amounts of change in the resistance value upon external force application or moisture dropping differ by 5 times or more, and the output changes moment by moment as moisture dries, it is possible to distinguish between the different detections based on the behavior of the output values. With this embodiment it is possible to simultaneously detect the amount of human body sweat or its time-dependent change, or water leakage, or urine leakage.

Alternatively, it may be in a form in which application of external force is detected but the sensor sensitivity is orders of magnitude lower (essentially no sensor sensitivity ) for contact of fluid such as moisture or sweat. For example, the cover yarn material may be a material with higher conductivity than the conductivity of a fluid which is not to be detected, thereby providing high sensor sensitivity for application of external force while making the sensor insensitive to contact of the fluid. For example, by using fiber with a carbon-based conductive material in at least part of the surface for high resistance fiber as the covering material (sheath yarn), it is possible to read off change in resistance between linear conductors, to detect changes in body respiration or posture or movement of the wrist or fingers, without producing change in sensor output in response to sweat or water leakage. The change in output of the sensor in response to change in moisture content at the parts that measure biometric information is preferably 1/20 or lower and more preferably 1/100 or lower with respect to the change in output of the sensor with respect to the measurement of biometric information, such as external force, which is to be detected.

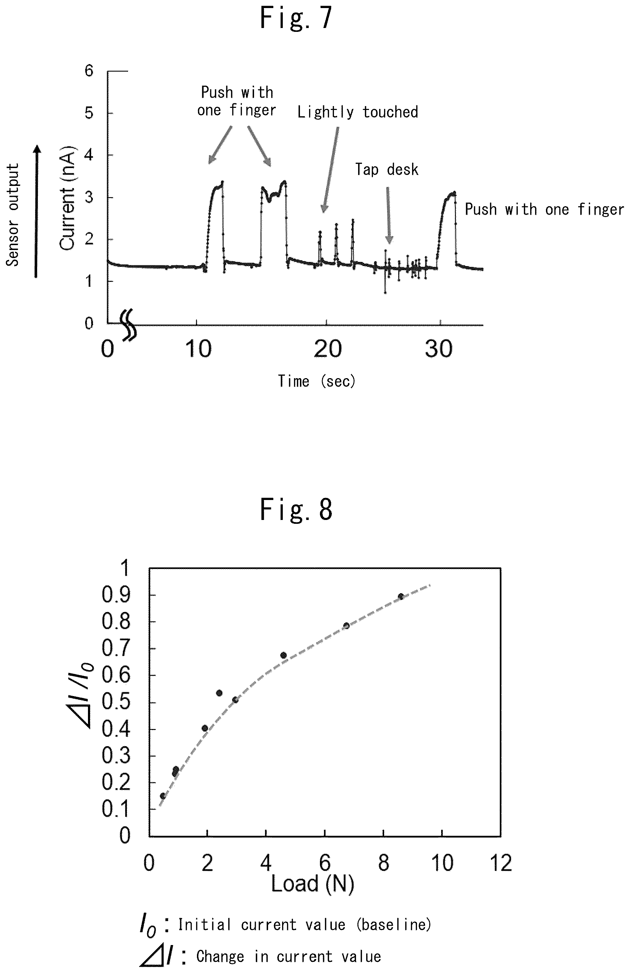

Fig. 7 shows, as an example of a sensing member of the invention, construction of double core-sheath structure yarn using conductive fiber composed of multifilaments of silver plated nylon 66 fiber, as a linear conductor, and using insulating fiber made of polyester, as sheath yarn, to construct a double core-sheath structure yarn, and using a plied structural yarn of two double core-sheath structure yarns twisted together, as an example of measuring load sensing characteristics. For this example, the fineness of the nylon fiber was 220 dtex, the fineness after silver plating was 300 dtex, and the linear conductor had 68 filaments. As the sheath yarn covering conditions, 2 bobbins of wooly yarn of 252 dtex/108 filament polyester were used for the sheath yarn, Z-twisting each with a twisting number Z of 732 T/m. Further twisting together two of the obtained double core-sheath structure yarns produced plied structural yarn twisted in the S direction, with a twisting number S of 170 T/m. The fineness of the plied structural yarn was 2000 dtex. The twist coefficient K of the plied structural yarn was calculated to be $(300 + 252 \times 2)^{1/2} \times 732 = 20{,}756$.

The resistance value between two linear conductors of two adjacent core-sheath structure yarns was measured by electrically opening the two linear conductors at the ends of the core-sheath structure yarns, and connecting a source meter (SMU: source major unit, Model 2614B by Keithley Co.) which allowed simultaneous measurement of voltage, current and resistance upon supply of voltage and current between the two linear conductors on the other ends. A voltage of 3 V was applied between the two linear conductors, and the current value before and after application of load or tensile force was measured using a proprietary program for constant monitoring of the current value outputted by the source meter. For plied structural yarn, the sensing property was measured using a sample fabricated so that the length of the paired linear conductor section (the effective length for sensing) was 10 cm.

Fig. 8 shows the relationship between applied load and rate of change in current value, upon application of a load to the obtained plied structural yarn. The measuring conditions for the current value were exactly the same as in Fig. 7. Load application was measured by placing the sensing fiber member on a flat stage and applying a load using a force gauge (Full-Range 20N by Imada Co.), while monitoring the load value. A circular indenter with a diameter of φ12.5 mm was used. The results shown in Fig. 8 demonstrate that the plied structural yarn is useful as a sensing fiber member that can also detect the magnitude relationship of loads.

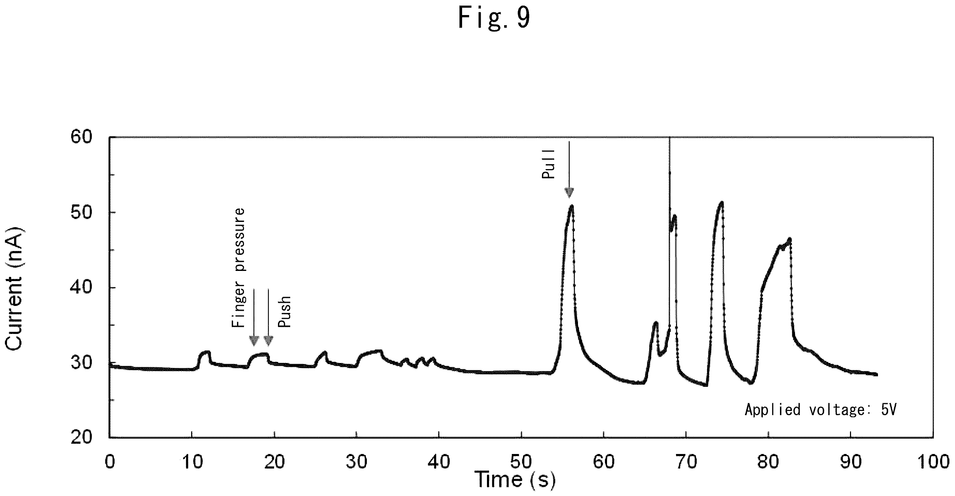

As an example of a woven fabric using the obtained core-sheath structure yarn, five of the aforementioned core-sheath structure yarns as warp yarns and one as weft yarn were woven in the woven fabric structure shown in Fig. 5 to create a narrow woven fabric with 1 cm width × 10 cm length, and a thickness of 850 μm. Fig. 9 shows the time-dependent change in current value (sensor output) at a section near the intersection of the core-sheath structure yarns in a woven fabric when it was subjected to the load of a finger and then to tensile force. It was thus shown that the woven fabric is useful as a sensing fiber member to detect load or tensile force.

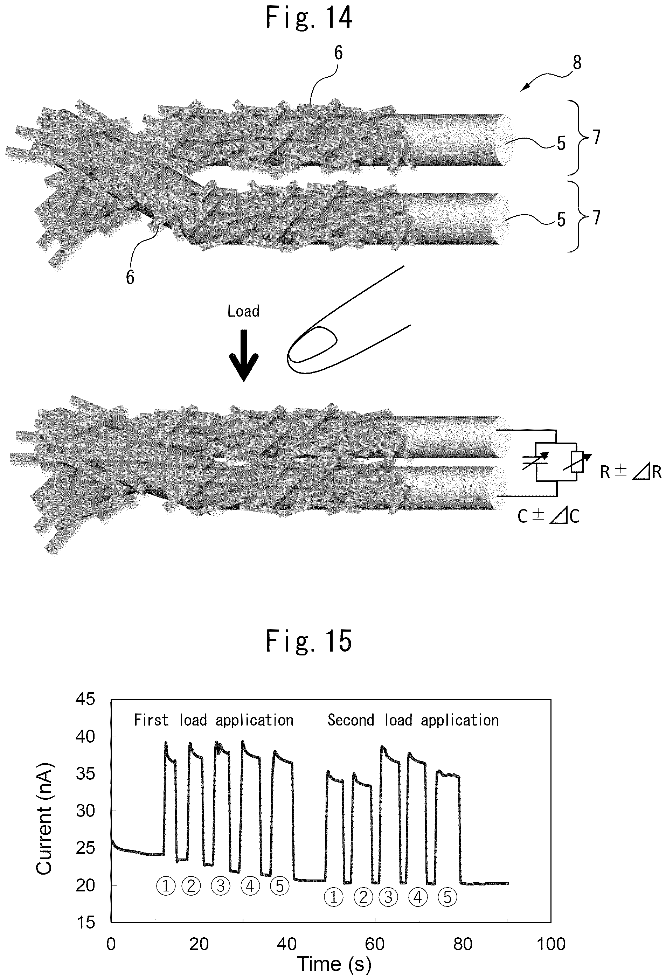

Fig. 14 shows a conceptual drawing for an example of a sensing member with CSY as sheath yarn, as an example of the sensing member of the invention. The sensing yarn can be produced at drastically lower cost than conventional piezoelectric sensing yarn which is provided with electrodes on the inner side and outer side in contact with a piezoelectric element as shown in Fig. 13.

As an example, Fig. 15 shows sensing properties for a sensing member of plied structural yarn obtained by CSY processing using conductive fiber composed of multifilaments of silver-plated nylon 66 fibers, as the linear conductor, and using a cupra sliver (product of Asahi Kasei Corp., thread size: 10/1) for the sheath yarn, and further twisting together two of the obtained core spun yarns. Using a thread with a nylon fiber fineness of 33 dtex, a post-silver plating fineness of 40 dtex and a filament count of 7 as the linear conductor and Micro-Lyocell (product of Lenzing, "Lyocell Micro", thread size: 15/1) as the sheath yarn, a VORTEX spinning machine by Murata Machinery Co. was used for CSY (core spun yarn) processing at a spinning speed of 300 m/min. The core yarn/sheath yarn weight mixing ratio was 8/92 (core yarn mixing ratio: 8 wt%). The two obtained core spun yarns were combined and further S-twisted to produce plied structural yarn with a twisting number of 163 T/m. The fineness of the plied structural yarn was 1180 dtex. Fig. 15 shows the state of change in the current value (sensor output) upon application of a load to the obtained plied yarn. After twice carrying out an operation of 5 repetitions of application and removal of a load at different locations, the change in current value taking place with application and removal of the load at each measurement point was confirmed. Fig. 15 shows that the sensing member is able to detect a load.

The following Examples employ a human body respiration measuring device, a respiration/posture simultaneous measuring device, a finger bending measuring device and a hand opening/closing and wrist bending measuring device, as biometric information measurement devices according to the embodiment.

EXAMPLES

The invention will now be explained in greater detail using Examples, with the understanding that the invention is in no way limited by the Examples.

The specific load elongations for the sensing members and clothing in the Examples were measured in the following manner.

<Specific load elongation (%)>

A fabric sample was cut to a width of 1 cm and a length of 25 cm and attached to a tensile tester with chucking of 5 cm at each end, applying an initial tension of 10 g (0.1 N) to a test length of 15 cm.

A process of stretching at a pull rate of 80 mm/min to a load of 1 N followed by restoration was repeated 75 times. The amount of elongation was measured at each stretching step, and the ductility at the 75th elongation (elongation rate with respect to the initial length) was defined as the specific load elongation according to the invention.

[Example 1: Sensing of respiration using narrow woven fabric with embedded sensing yarn]

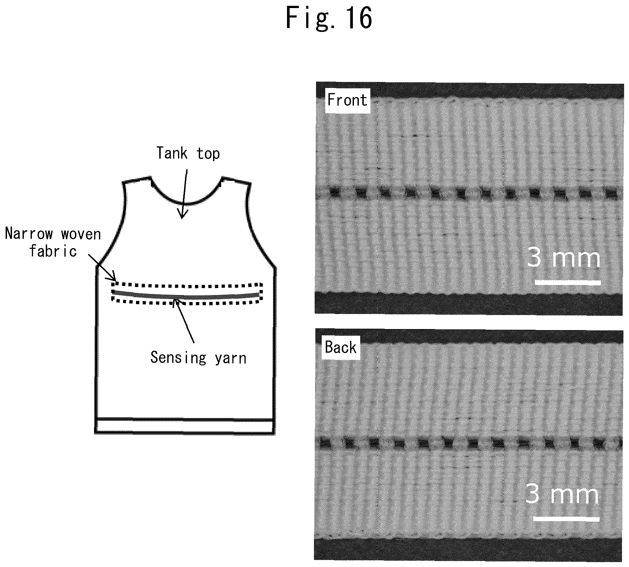

Sensing wear prepared by sewing a narrow woven fabric with interwoven sensing yarn (sensing member) on the back side of a tank top, as shown at left in Fig. 16, was worn by a person and used for respiration sensing. The location of the sensing yarn was adjusted while sensing wear was being worn so that the sensing yarn was near the diaphragm, i.e. near the 4th to 5th rib from the bottom. The tank top fabric used was 60% cotton, 30% polyester, and 10% polyurethane, with a size that fit snugly to the body. In other words, the wear was designed for close fitting between the body surface and the sensing wear.

The linear conductor in the sensing yarn was conductive fiber comprising multifilaments of silver-plated nylon-6,6 fiber, with a nylon fiber fineness of 66 dtex, a silver-plated fineness of 80 dtex, and a filament count of 14. The linear conductor was covered with Beltron B31$^R$ (fineness: 240 dtex) by KB Seiren Co., Ltd. as high resistance fiber for the covering material (sheath yarn), to prepare core-sheath structure yarn, two of which were combined and further twisted to obtain plied structural yarn as sensing yarn. The fineness of the sensing yarn was 1284 dtex. The covering conditions for production of the plied structural yarn were first double covering using two sheath yarns with a twist count of 653 T/m in the Z direction (twist coefficient = 15,258), and then with a twist count of 250 T/m in the S direction (twist coefficient = 8344) when the two sheath-core structures were further twisted together. A narrow woven fabric was produced having the sensing yarn interwoven at the center section of the warp yarn of the narrow woven fabric (woven fabric tape). The woven structure was a plain weave, and polyester woolly yarn with a fineness of 167T was used for both the warp yarn and weft yarn at the sections of the narrow woven fabric other than the sensing yarn. The obtained narrow woven fabric had a width of 10 mm, a thickness of 430 μm, and a weight of 2.14 g per 1 m woven fabric length. A magnified view of the narrow woven fabric used is shown at right in Fig. 16. The black sections appearing at the center of the photograph are the sensing yarn, interwoven into the narrow woven fabric in a plain weave structure. A 25 cm-long piece of the sensing narrow woven fabric was sewn onto the back side of a tank top to produce the sensing wear shown at left in Fig. 16. The specific load elongation of the tank top fabric was 9%, and the specific load elongation of the narrow woven fabric was 1.8%.

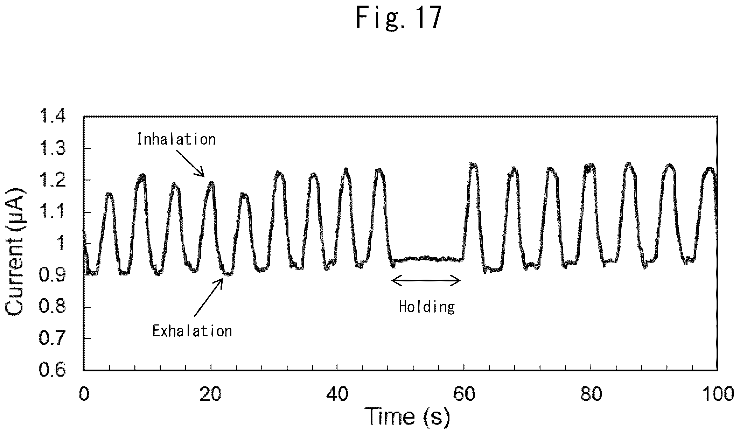

The respiration sensing properties were evaluated in the following manner. Two adjacent linear conductors were monitored for output current with one end of the core-sheath structure yarn left electrically open and a voltage of 1.0 V applied at the other end. The voltage application and current value were measured using a source meter (SMU: Source Major Unit 2614B, product of Keithley), and the output current value was constantly monitored using a proprietary program. The results are shown in Fig. 17. Based on the results shown in Fig. 17, the output value was found to fluctuate and the measured change correlated with respiration, with the output current value increasing during breathing movement and decreasing during exhalation movement. The output current value was constant when the breath was held. The sensing wear can thus be used to show changes in sensor output correlating with respiration. The change in the length of the body surface in response to respiration was about 3.9% at a 25 cm-long area on the back side of the human body on which the sensing yarn was fitted. The measurement was made while worn by a female with a body height of 152 cm.

[Example 2: Simultaneous sensing of respiration and posture using narrow woven fabric with embedded sensing yarn]

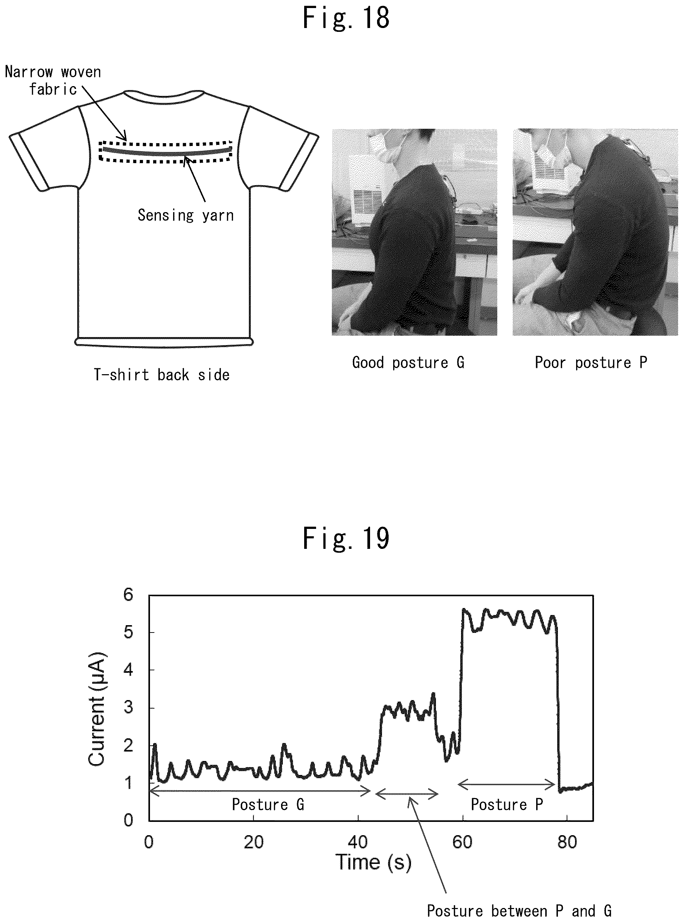

A sensing yarn-interwoven narrow woven fabric similar to Example 1 was prepared, and a T-shirt having it sewn at a location near the scapula was produced as sensing wear (left in Fig. 18). The fabric used for the T-shirt was 89% polyester and 11% polyurethane. The specific load elongation of the T-shirt fabric was 11%, and the specific load elongation of the narrow woven fabric was 1.8%.

The sensing wear was worn, and the current value was measured during movement under the same conditions as Example 1, evaluating good posture with straightened spine as "G" and a poor hunching posture as "P", as shown at right in Fig. 8.

Fig. 19 shows an example of the results of monitoring the current value. The subject was a male with a body height of 178 cm. The subject had good posture up to about 0 to 42 s, with slight hunching between 42 to 55 s, followed by return to a good posture, and poor posture with significant hunching for 60 to 78 s thereafter, as shown in the photograph at right in Fig. 18. The results in Fig. 19 demonstrate that the baseline level for the current value correlated with poorness of posture, while fluctuation in the current value correlated with respiration regardless of the state of posture. Comparison with the results of separately measuring the respiration rate from rib movement confirmed that the changes in output current shown in Fig. 19 fully correlated with exhalation and inhalation.

[Example 3: Sensing of finger bending with finger supporter/glove]

The linear conductor forming the sensing yarn was conductive fiber composed of a multifilament of nylon-6,6 fiber with silver plating. The fineness of the nylon fiber was 66 dtex, the fineness after silver plating was 80 dtex, and the linear conductor had 14 filaments. The linear conductor was covered with Bemberg[R] (fineness: 220 dtex) by Asahi Kasei Corp. as high resistance fiber for the covering material (sheath yarn), to prepare core-sheath structure yarn, two of which were combined and further twisted to obtain plied structural yarn as sensing yarn. The fineness of the sensing yarn was 1255 dtex. The covering conditions for production of the plied structural yarn were double covering using two sheath yarns with a twist count of 1042 T/m in the Z direction (twist coefficient = 23,439), and with a twist count of 204 T/m in the S direction (twist coefficient = 6490) when the two sheath-core structures were further twisted together.

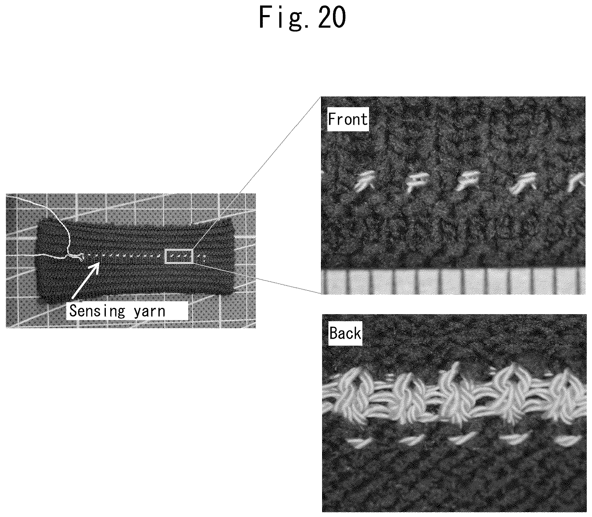

A whole garment flat knitting machine (MACH2XS 15S) was used to fabricate a finger supporter using the sensing yarn shown in Fig. 20. Using 168 dtex/36f polyester woolly yarn for the yarn forming the front side of the supporter and for the ground thread forming the back side, a structure was formed having holes at both ends in the horizontal direction of knitted sections for insertion of the fingers (tubes extending in the wale direction of the knitted fabric), obtained by knitting together the yarns on the front side and back side. Knitting was with 46 courses on both the front side and back side, for a total of 92 courses in one round, extending for 60 wales in the lengthwise direction to form the structure shown in Fig. 20. The loop length was 5.5 mm.

For knitting, the sensing yarn was knitted into the back of the center of the front side, by needle bag knitting, with 4 courses in the widthwise direction of the tube and 38 wales at a loop length of 5 mm in the lengthwise direction.

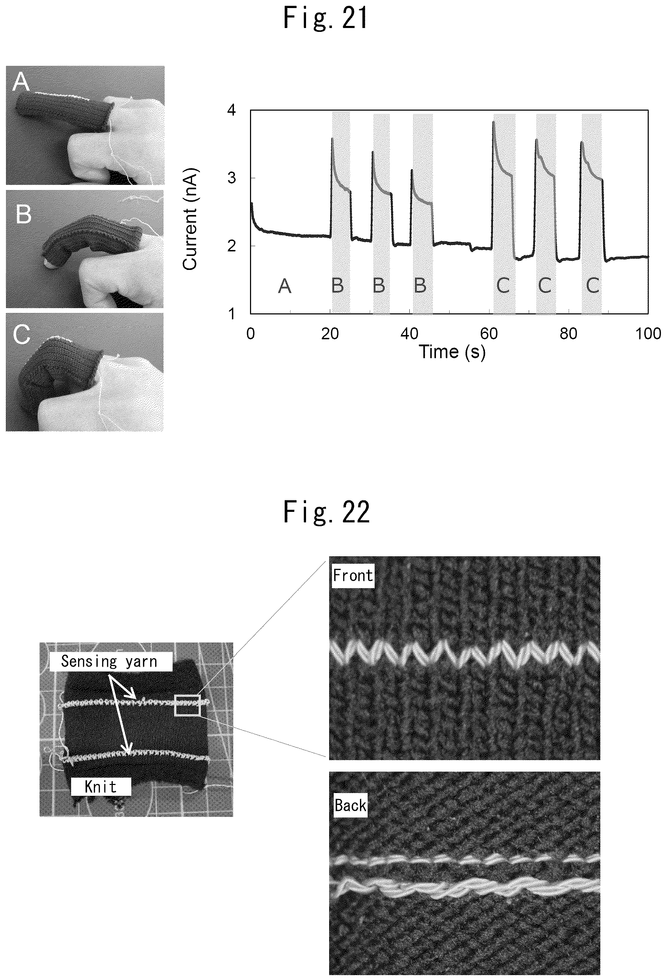

The finger supporter (see Fig. 20) was fitted onto the middle finger as shown at left in the photographs of Fig. 21, and the sensor output current value was monitored while bending and straightening the finger by the same measuring method as in Example 1, except that the applied voltage was 3.0 V. The results are shown at right in Fig. 21. Movements B and C were made at the times corresponding to the hatch-shaded portions marked as B and C, with the finger extended outward as in A at all other times. Based on the right-hand graph in Fig. 21, the sensor output current increased when the finger was bent, with greater change in current value the larger the degree of bending. While some overshoot in the current value was seen with the initial movements B and C, this was due to relaxation of the structure, since the knitted structure of this Example was somewhat less able to follow shape deformation, whereas higher responsiveness was obtained with the different knitted structure used in Example 4 below. In other words, the sensing yarn itself exhibited high responsiveness. The lengths of the outer surface of the finger during bending and extension movement were measured and found to be 4.0 cm in the extended state A, 4.8 cm in state B and 5.0 cm in state C. In other words, states B and C had extensions of 20% and 25%, respectively, compared to the reference state A. This indicated stretching of the sensing yarn in the finger supporter in response to extension of the finger surface when in the bent state, which increased the current value.

[Example 4: Simultaneous sensing of opening and closing of the hand and wrist bending, using a wristband]

The linear conductor forming the sensing yarn was conductive fiber composed of a multifilament of nylon-6,6 fiber with silver plating. The fineness of the nylon fiber was 66 dtex, the fineness after silver plating was 80 dtex, and the linear conductor had 14 filaments. The linear conductor was covered with Bemberg[R] (fineness: 220 dtex) by Asahi Kasei Corp. as high resistance fiber for the covering material (sheath yarn), to prepare core-sheath structure yarn, two of which were combined and further twisted to obtain plied structural yarn as sensing yarn. The fineness of the sensing yarn was 1255 dtex. The covering conditions for production of the plied structural yarn were first double covering using two sheath yarns with a twist count of 1042 T/m in the Z direction (twist coefficient = 23,439), and then with a twist count of 204 T/m in the S direction (twist coefficient = 6490) when the two sheath-core structures were further twisted together.

A whole garment flat knitting machine (MACH2XS 15S) was used to fabricate a wristband using the sensing yarn shown in Fig. 22. 168T72 polyester woolly yarn was used as the ground thread in the wristband main body, and a structure was formed having a hole for the wrist at both ends in the direction perpendicular to the knitted section (a tube extending in the course direction of the knitted fabric). Knitting was with a length of 96 courses on both the front side and back side, and 52 wales at the front and rear for a total of 104 wales as one round, to form the structure shown in Fig. 22. The loop length was 6 mm.

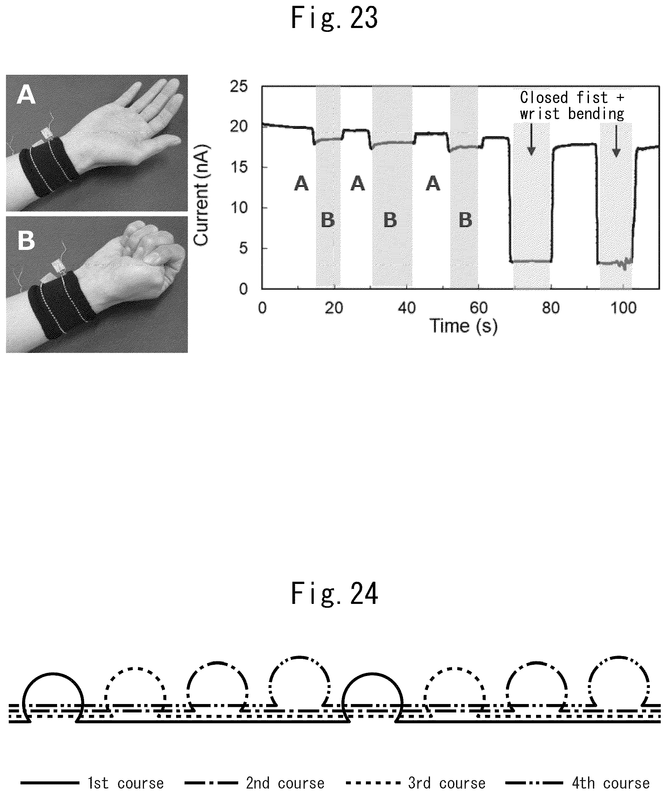

During knitting, two rows of sensing yarn running parallel in the lengthwise direction of the tube were knitted by smooth weaving to form one course from knitting of 4 courses, as shown in Fig. 24.

The wristband (see Fig. 22) was fitted onto the wrist as shown at left in the photographs of Fig. 23, and the sensor output current value was monitored while bending and straightening all five fingers by the same measuring method as in Example 1, except that the applied voltage was 3.0 V. The results are shown at right in Fig. 23. As seen in the left-hand photographs of Fig. 23, with the open-palm state as A, the closed fist state as B and the closed fist state with bent wrist as C, movements B and C were carried out at the times corresponding to the hatch-shaded portions marked as B and C in the graph at right in Fig. 23, returning to the open-palm state A at all other times. Based on the right-hand graph in Fig. 23, the sensor output current increased when the fingers were bent, with greater change in current value the larger the degree of bending. While some overshoot in the current value was seen with the initial movements B and C, this was due to relaxation of the structure, since the knitted structure of this Example was somewhat less able to follow shape deformation, whereas higher responsiveness was obtained with the different knitted structure used in Example 4. In other words, the sensing yarn itself exhibited high responsiveness. The length around the wrist during movement was measured and found to be 18.0 cm in the open-palm state A, 17.7 cm in state B and 17.0 cm in state C. In other words, states B and C produced contraction of 1.7 % and 5.6 %, respectively, compared to the reference state A. The output current value during movements B and C was reduced, allowing detection of hand clenching movement due to contraction of the initially stretched sensing yarn.

[Example 5]

The linear conductor forming the sensing yarn was conductive fiber composed of a multifilament of nylon-6,6 fiber with silver plating. The fineness of the nylon fiber was 99 dtex, the fineness after silver plating was 120 dtex, and the linear conductor had 21 filaments. Sheath-core structure yarn was produced using KURACARBO KC-782R B20T4 by Kuraray Co., Ltd.) with a fineness of 500 dtex, as high resistance fiber for the covering material (sheath yarn), and two of these were further twisted together to form plied structural yarn, three yarns of which were directly sewn into a tank top. Respiration sensing wear was produced in the same manner as Example 1 as regards the other conditions. The twist count during covering of the sheath yarn was 838 T/m (twist coefficient = 28,045), the twist count for further twisting of two of the sheath-core structure yarns was 280 T/m (twist coefficient = 13,252), and the fineness of the completed plied structural yarn was 2865 dtex. The sewing locations were three locations: the upper chest region (location just below the armpits), the lower chest region (the underbust location), and the abdominal region (location of greatest movement during abdominal breathing), and sewing was with a lock sewing machine. The sensor resistance of sensing yarn with a length of 30 cm was 9.0 kΩ, and the wiring resistance value was 23 Ω per yarn, as the full core yarn of the linear conductor. As a result of measuring the sensor resistance value after thoroughly soaking the sensing yarn with tap water and synthetic sweat, it was found to be 9.0 kΩ, which was exactly the same as when dry.

When the sensing wear was worn and the sensor output was read off, the same changes in sensor output corresponding to respiration were seen. Thoracic breathing and abdominal breathing produce greater changes in the upper chest, lower chest or abdominal region, allowing distinction to be made between thoracic and abdominal breathing. Even when the sensing member was soaked with tap water, it was still possible to obtain the same sensor output as when dry. The measurement was made while worn by a male with a body height of 175 cm.

INDUSTRIAL APPLICABILITY

A sensing member used in the biometric information measurement device of the invention can be processed to long lengths, has excellent mass productivity, allows use as warp yarn for woven fabrics or warp knitting and is pliable with an excellent feel, while it can also be provided at much lower cost than contact sensing fiber members (piezoelectric threads) for contact using conventional piezoelectric materials. Specifically, since the sensing member allows sensing of a load or tensile force using the common fiber materials polyester or nylon, it is possible to obtain contact sensing fibers at very low cost, and since it includes core spun yarn and employs established covering technology for processing of the fibers, it is possible to achieve processing to long lengths and to obtain excellent mass productivity, while also obtaining processed yarn with a highly superior feel compared to piezoelectric yarn, thereby facilitating formation into fiber members such as woven or knitted fabrics.

The sensing member used in the biometric information measurement device of the invention varies in electrostatic capacitance and/or resistance, and can thereby detect the state of continuous application of a load or tensile force. It is also able to detect changes in moisture content.

With the sensing member used in the biometric information measurement device of the invention, if the core-sheath structure yarn having high resistance staple fiber as a covering material wrapped in random directions around a linear conductor as the core material to cover it consists of core spun yarn, then it will be easier to use natural fiber and biodegradable yarn, and easier to impart functionality to the sheath yarn.

The biometric information measurement device of the invention therefore has a wide range of utility for use in smart textiles having an electrical functional element provided on a flexible and stretchable fiber base material.

Sensing members of the prior art utilize sensor output such as electrical resistance and electrostatic capacitance in response to elongation of stretchable wiring, which depends primarily on the physical properties of the rubber elastomer, while exhibiting poor linearity and hysteresis as well as slow responsiveness when returned to the original state, and consequently the repeatability has been less than satisfactory. In contrast, the sensing member used in the biometric information measurement device of the invention has lower specific load elongation than a conventional sensing member, and therefore exhibits satisfactory linearity, virtually no hysteresis, fast responsiveness when restored to the original state, and satisfactory repeatability.

With the biometric information measurement device of the invention, therefore, it is possible to detect displacement or movement such as slight stretching of the body surface, or fluctuation in moisture content, using wear (such as a T-shirt, underwear, vest or belt) which includes a sensing fiber member (yarn) having plied structural yarn with two sheath-core structure yarns that are further twisted together, and thereby to detect changes in respiration or posture, movement of the wrist, ankles or fingers, movement of muscles (such as pelvic floor muscles, brachioradialis muscle or calf muscles), to analyze swallowing motion, walking or running motion (for rehabilitation or preventive care purposes), or to detect fluctuation in moisture content due to sweating or excretion. In particular, the biometric information measurement device of the invention allows simultaneous detection of the state of respiration and quality of posture, and also simultaneous detection of opening and closing of the hand (fist and open palm) or bending of the wrist.

The biometric information measurement device of the invention can also be designed so that posture-improvement inner wear communicates with an external device such as a smartphone using wireless communication such as Bluetooth$^R$, allowing its application as a smart textile which displays the number of proper postures per day together with a target value, based on a sensor in the dorsal region.

REFERENCE SIGNS LIST

1 Conductive fiber
2 Piezoelectric material
3 Conductor
4 Prior art piezoelectric yarn
5 Linear conductor as core material
6 High resistance fiber (filament or spun yarn) as covering material (cover yarn)
7 Core-sheath structure yarn
8 Plied structural yarn comprising two core-sheath structure yarns further twisted together
9 Core yarn (core material)
10 Spindle
11 Bobbin
12 Flyer
13 Flyer cap
14 Cover yarn
15 Flyer drive guide
16 Flyer drive guide
17 Flyer drive guide
18 Flyer drive guide

**Claims**

1. A biometric information measurement device which comprises a fiber base material that includes a sensing member and which is disposed in contact with a predetermined position of the body, wherein the sensing member has at least two core-sheath structure yarns comprising high resistance fiber arranged as covering material around a linear conductor as the core material, two of which are disposed in mutual proximity, and is able to read off the change in resistance and/or change in capacitance between linear conductors of two core-sheath structured yarns, and based on that change, measures biometric information or fluctuation in the same.

2. The biometric information measurement device according to claim 1, wherein the sensing member is plied structural yarn having two core-sheath structure yarns further twisted together.

3. The biometric information measurement device according to claim 1 or 2, wherein the high resistance fiber arranged in the sensing member is made of fiber having a carbon-based conductive material provided on at least part of the surface, and is able to read off change in resistance between the linear conductors.

4. The biometric information measurement device according to claim 1 or 2, wherein the specific load elongation of the sensing member is 5% or lower.

5. The biometric information measurement device according to claim 1 or 2, wherein the biometric information is change in body posture.

6. The biometric information measurement device according to claim 1 or 2, wherein the biometric information is related to respiration.

7. The biometric information measurement device according to claim 1 or 2, wherein the biometric information is change in moisture content of a biological surface.

8. The biometric information measurement device according to claim 3, wherein the change in output of a sensor with respect to change in moisture content at the section where the sensing member measures biometric information is 1/20 or lower with respect to change in sensor output for biometric information measurement.

9. The biometric information measurement device according to claim 1 or 2, wherein at least one of the core-sheath structure yarns is a core-sheath structure yarn wherein high resistance fiber as a covering material is wrapped in one direction around a linear conductor as the core material, covering it.

10. The biometric information measurement device according to claim 1 or 2, wherein at least one of the core-sheath structure yarns is a core spun yarn wherein high resistance staple fiber as a covering material is wrapped in random directions around a linear conductor as the core material, covering it.

11. The biometric information measurement device according to claim 1, wherein two or more of the core-sheath structure yarns are disposed in partial contact in the fiber base material.

12. Clothing in which a biometric information measurement device according to claim 1 or 2 is disposed.

13. Clothing in which a biometric information measurement device according to claim 11 is disposed.

# Fig. 1

# Fig. 2

Fig. 3

Fig. 4

# Fig. 5

# Fig. 6

Two core-sheath structure yarns

Output monitor program

About 1~5 V

GND

Source meter
(SMU)

PC

# Fig. 7

# Fig. 8

$I_0$ : Initial current value (baseline)

$\triangle I$ : Change in current value

Fig. 9

# Fig. 10

Part B

14
16
15
13
17
12
18
11
10
9

# Fig. 11

14
9

# Fig. 12

Measured object
Equivalent circuit

Capacity component

C

Rp

Resistance component

External resistance, contact resistance, etc.

Rs

Impedance measuring circuit

Power supply

A: Ammeter

V: Voltmeter

# Fig. 13

4

+

−

3

2

1

EP 4 544 999 A1

# Fig. 14

# Fig. 15

30

# Fig. 16

EP 4 544 999 A1

Fig. 17

# Fig. 18

Narrow woven fabric

Sensing yarn

T-shirt back side

Good posture G

Poor posture P

# Fig. 19

Posture G

Posture P

Posture between P and G

# Fig. 20

# Fig. 21

# Fig. 22

# Fig. 23

# Fig. 24

―――――― 1st course　――・―― 2nd course　―・・・・・― 3rd course　――・・―― 4th course

## INTERNATIONAL SEARCH REPORT

International application No.

**PCT/JP2023/022982**

### A. CLASSIFICATION OF SUBJECT MATTER

*A61B 5/107*(2006.01)i; *A61B 5/00*(2006.01)i; *A61B 5/0533*(2021.01)i; *A61B 5/113*(2006.01)i; *H10N 30/30*(2023.01)i; *H10N 30/857*(2023.01)i

FI: A61B5/107 300; A61B5/00 M; A61B5/0533; A61B5/113; H10N30/30; H10N30/857

According to International Patent Classification (IPC) or to both national classification and IPC

### B. FIELDS SEARCHED

Minimum documentation searched (classification system followed by classification symbols)

A61B5/107; A61B5/00; A61B5/0533; A61B5/113; H10N30/30; H10N30/857

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2010-014694 A (NISSAN MOTOR CO., LTD.) 21 January 2010 (2010-01-21) paragraphs [0011]-[0341] | 1-5, 11-13 |
| Y | | 6-10 |
| Y | JP 2008-210557 A (KURARAY CO., LTD.) 11 September 2008 (2008-09-11) paragraphs [0017]-[0084] | 6 |
| Y | US 2016/0186366 A1 (FOOTFALLS AND HEARTBEATS LTD.) 30 June 2016 (2016-06-30) paragraphs [0048]-[0220] | 6-8 |
| Y | US 2002/0194934 A1 (TAYLOR L. Geoffrey) 26 December 2002 (2002-12-26) paragraphs [0029]-[0054] | 9 |
| Y | US 2006/0258247 A1 (THE HONG KONG POLYTECHNIC UNIVERSITY) 16 November 2006 (2006-11-16) paragraphs [0028]-[0080] | 10 |

☐ Further documents are listed in the continuation of Box C.　　☑ See patent family annex.

| | |
|---|---|
| *　　Special categories of cited documents: | "T"　later document published after the international filing date or priority date and not in conflict with the application but cited to understand the principle or theory underlying the invention |
| "A"　document defining the general state of the art which is not considered to be of particular relevance | |
| "E"　earlier application or patent but published on or after the international filing date | "X"　document of particular relevance; the claimed invention cannot be considered novel or cannot be considered to involve an inventive step when the document is taken alone |
| "L"　document which may throw doubts on priority claim(s) or which is cited to establish the publication date of another citation or other special reason (as specified) | "Y"　document of particular relevance; the claimed invention cannot be considered to involve an inventive step when the document is combined with one or more other such documents, such combination being obvious to a person skilled in the art |
| "O"　document referring to an oral disclosure, use, exhibition or other means | |
| "P"　document published prior to the international filing date but later than the priority date claimed | "&"　document member of the same patent family |

| Date of the actual completion of the international search | Date of mailing of the international search report |
|---|---|
| **18 August 2023** | **05 September 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
**Information on patent family members**

| International application No. |
| --- |
| **PCT/JP2023/022982** |

| Patent document cited in search report | | | Publication date (day/month/year) | Patent family member(s) | Publication date (day/month/year) |
| --- | --- | --- | --- | --- | --- |
| JP | 2010-014694 | A | 21 January 2010 | (Family: none) | |
| JP | 2008-210557 | A | 11 September 2008 | (Family: none) | |
| US | 2016/0186366 | A1 | 30 June 2016 | WO 2015/022671 A1 p. 8, line 1 to p. 52, line 5 | |
| US | 2002/0194934 | A1 | 26 December 2002 | (Family: none) | |
| US | 2006/0258247 | A1 | 16 November 2006 | (Family: none) | |

Form PCT/ISA/210 (patent family annex) (January 2015)

# EP 4 544 999 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 6025854 B
- JP 6689943 B
- JP 2020090768 A
- JP 2020036027 A
- JP 6107069 B
- JP 5754946 B
- JP 2006234716 A
- JP 2016173685 A
- JP 10025635 A
- JP 2013231246 A
- JP 2018507081 A
- JP 2011098214 A
- JP 6960725 B
- JP 698667 A
- JP 2018021270 A
- JP 2014025180 A
- JP 2017123911 A
- JP 2016509635 A
- JP 2017512542 A
- JP 2019072048 A
- JP 2022055855 A
- WO 2018056062 A
- US 4444205 A
- US 20160113581